# EUROPEAN PATENT APPLICATION

(11) **EP 4 626 191 A1**
(43) Date of publication of application: **01.10.2025**
(21) Application number: 23894221.3
(22) Date of filing: 31.08.2023
(51) Int. Cl.: H10K 30/60, C07D 471/04, C07D 487/04, H01L 27/146, H10K 30/30, H10K 30/50, H10K 30/86, H10K 39/32, H10K 85/60

(54) **ORGANIC ELECTRONIC ELEMENT AND COMPOUND**

(30) Priority: 21.11.2022 JP 2022185686
(71) Applicant: Tosoh Corporation, Yamaguchi 746-8501 (JP)
(72) Inventor: SHINYA, Hirokazu, Ayase-shi, Kanagawa 252-1123 (JP); ARAI, Nobumichi, Ayase-shi, Kanagawa 252-1123 (JP); OKADA, Takeshi, Ayase-shi, Kanagawa 252-1123 (JP); OHTA, Eriko, Ayase-shi, Kanagawa 252-1123 (JP)
(74) Representative: Müller-Boré & Partner Patentanwälte PartG mbB
(86) International application number: PCT/JP2023/031748
(87) International publication number: WO 2024/111206

(57) **Abstract**

Provided is an organic electronic element that has an improved hole transport ability and a compound that is used in the organic electronic element and that can improve the hole transport ability thereof. The organic electronic element comprises a first electrode, a second electrode and an organic layer disposed between the first electrode and the second electrode. The organic layer includes a hole transport layer and a hole transport promoting layer containing a compound having a partial structure represented by the following formula (1), or includes a layer comprising a mixture of a hole transport material and the compound having the partial structure represented by formula (1), and the organic electronic element includes a light-receiving layer: 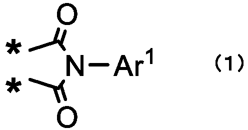 (wherein * represents a bond, and formula (1) forms a cyclic imide structure; Ar¹ represents a monocyclic or fused-ring aromatic hydrocarbon group optionally substituted with a substituent or a monocyclic or fused-ring heteroaromatic group optionally substituted with a substituent, the aromatic hydrocarbon group may be a group in which a plurality of aromatic hydrocarbon groups are linked together directly or with a linking group therebetween, the heteroaromatic group may be a group in which a plurality of heteroaromatic groups are linked together directly or with a linking group therebetween, and Ar¹ may be a group in which the aromatic hydrocarbon group and the heteroaromatic group are linked together; and the substituent with which the aromatic hydrocarbon group or the heteroaromatic group is optionally substituted is a cyano group, a fluoro group, a chloro group, a bromo group, an iodo group, a trifluoromethyl group, a methyl group, a fluoroalkyl group having 2 to 10 carbon atoms, a fluoroalkoxy group having 1 to 10 carbon atoms or an alkyl group having 2 to 10 carbon atoms).

## Description

### Technical Field

The present invention relates to a compound having an imide skeleton as a partial structure and an organic electronic element including the compound.

### Background Art

Currently, attempts have been actively made to create new advanced devices using organic materials. In particular, research and development have been actively carried out on organic electronic elements such as photoelectric conversion elements and organic EL elements, and material and device designs for higher device performance have been developed. For example, photoelectric conversion elements for use in video capture have a problem in that slow transfer of carriers (electrons and holes) generated in a light-receiving layer to an electrode leaves a residual image. Accordingly, highly efficient transfer of carriers in an element is necessary for higher device performance.

An electronic material using an electron transport material containing a pyrazole derivative is disclosed (see PTL 1). The pyrazole derivative disclosed in PTL 1 is a pyrazole derivative having an imide skeleton as a partial structure.

However, there is a need for even higher performance in the field of organic electronic elements, and further improvements are necessary therefor.

### Citation List

### Patent Literature

PTL 1: Japanese Unexamined Patent Application Publication No. 2017-200912

### Summary of Invention

### Technical Problem

An object of the present invention is to provide an organic electronic element that is directed to a photoelectric conversion element and that has an improved ability to transport holes generated in a light-receiving layer, and also to provide a compound that is used in the organic electronic element and that can improve the hole transport ability thereof.

### Solution to Problem

The inventors have conducted intensive studies in order to solve the above problem and consequently have found that a compound having an imide skeleton as a partial structure can improve the hole transport ability of an organic electronic element directed to a photoelectric conversion element, thus completing the present invention.

Specifically, the present invention includes the following aspects:
[1] An organic electronic element comprising a first electrode, a second electrode and an organic layer disposed between the first electrode and the second electrode,
   wherein the organic layer includes a hole transport layer and a hole transport promoting layer containing a compound having a partial structure represented by the following formula (1), or includes a layer comprising a mixture of a hole transport material and the compound having the partial structure represented by formula (1), and
   the organic electronic element includes a light-receiving layer: 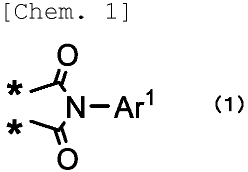 (wherein * represents a bond, and formula (1) forms a cyclic imide structure;
   Ar¹ represents a monocyclic or fused-ring aromatic hydrocarbon group optionally substituted with a substituent or a monocyclic or fused-ring heteroaromatic group optionally substituted with a substituent, the aromatic hydrocarbon group may be a group in which a plurality of aromatic hydrocarbon groups are linked together directly or with a linking group therebetween, the heteroaromatic group may be a group in which a plurality of heteroaromatic groups are linked together directly or with a linking group therebetween, and Ar¹ may be a group in which the aromatic hydrocarbon group and the heteroaromatic group are linked together; and
   the substituent with which the aromatic hydrocarbon group or the heteroaromatic group is optionally substituted is a cyano group, a fluoro group, a chloro group, a bromo group, an iodo group, a trifluoromethyl group, a methyl group, a fluoroalkyl group having 2 to 10 carbon atoms, a fluoroalkoxy group having 1 to 10 carbon atoms or an alkyl group having 2 to 10 carbon atoms).
[2] The organic electronic element according to [1], wherein the hole transport layer and the hole transport promoting layer are disposed adjacent to each other between the first electrode and the second electrode.
[3] The organic electronic element according to [1] or [2], wherein the compound having the partial structure represented by formula (1) is a compound represented by the following formula (2): 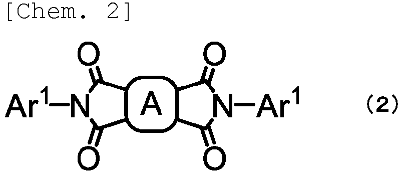 (wherein
   Ar¹ represents the same group as Ar¹ in formula (1), and one Ar¹ group is the same group as another Ar¹ group; and
   a ring A represents a monocyclic or fused-ring aromatic hydrocarbon ring, and the aromatic hydrocarbon ring may be a ring in which a plurality of aromatic hydrocarbon rings are linked together directly or with a linking group therebetween).
[4] The organic electronic element according to [3], wherein the ring A in formula (2) is represented by any of the following (A-1) to (A-9): 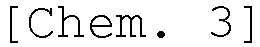 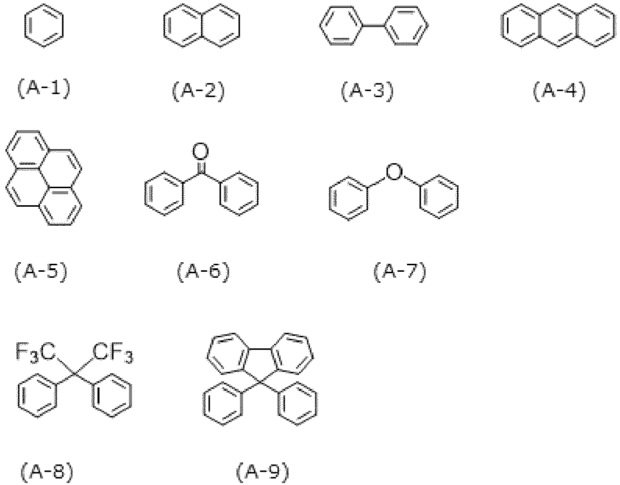
[5] The organic electronic element according to [4], wherein the ring A in formula (2) is formula (A-1) or formula (A-2).
[6] The organic electronic element according to any one of [1] to [5], wherein Ar¹ in formula (1) is an electron-withdrawing substituent.
[7] The organic electronic element according to any one of [1] to [6], wherein Ar¹ in formula (1) is a phenyl group, a pyridyl group, a pyrazyl group or a pyrimidyl group, each substituted with at least one group selected from the group consisting of a cyano group, a fluoro group and a trifluoromethyl group.
[8] An imide compound represented by the following formula (3): 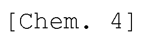 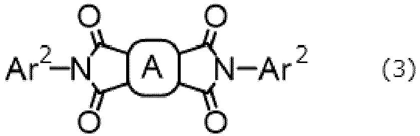 (wherein
   Ar² represents a pyridyl group, a pyrazyl group or a pyrimidyl group, each substituted with at least one group selected from the group consisting of a cyano group, a fluoro group and a trifluoromethyl group, and one Ar² group is the same group as another Ar² group; and
   a ring A represents any of the following (A-1) to (A-9) : 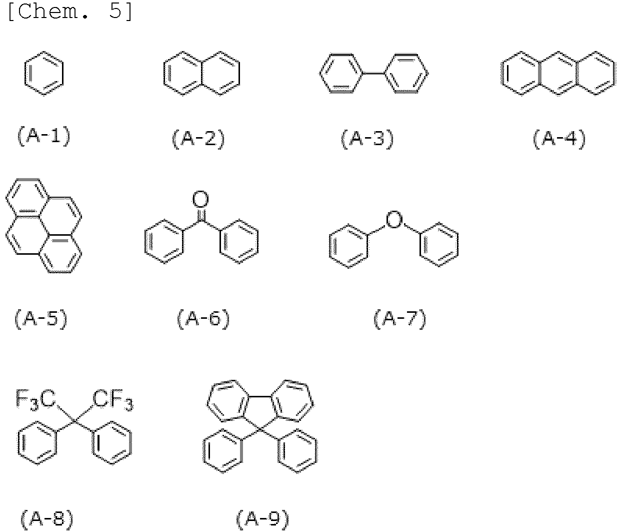
[9] The imide compound according to [8], wherein the ring A in formula (3) is formula (A-1) or formula (A-2).

### Advantageous Effects of Invention

The present invention can provide an organic electronic element that has an improved hole transport ability and a compound that is used in the organic electronic element and that can improve the hole transport ability thereof.

### Brief Description of Drawings

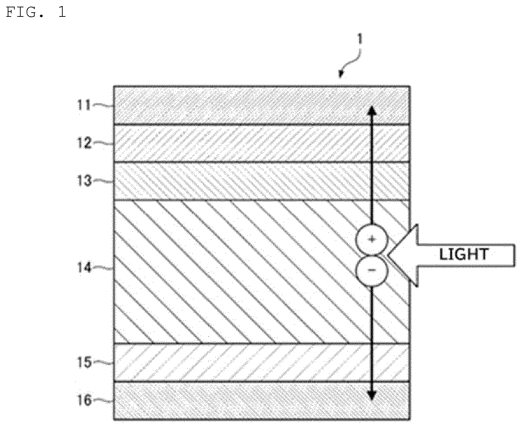

[Fig. 1] A schematic sectional view illustrating an example of a multilayer configuration of a photoelectric conversion element according to the present invention. Description of Embodiments

### (Organic Electronic Element)

An organic electronic element of the present invention is directed to a photoelectric conversion element including a light-receiving layer. The photoelectric conversion element is an element that converts light energy into electrical energy or electrical signals, and examples thereof include image capture elements, optical sensors and solar cells.

The organic electronic element of the present invention comprises a first electrode, a second electrode and an organic layer disposed between the first electrode and the second electrode.

The organic layer includes a hole transport layer and a hole transport promoting layer containing a compound having a partial structure represented by the following formula (1), or includes a layer comprising a mixture of a hole transport material and the compound having the partial structure represented by formula (1).

The organic electronic element includes a light-receiving layer.

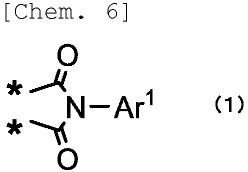

A detailed description of the compound having the partial structure represented by formula (1) above is given later.

The hole transport layer functions to transport holes and contains a hole transport material. The hole transport promoting layer is disposed between the first electrode and the hole transport layer, functions to allow smooth transfer of holes between the hole transport and the electrode and contains a hole transport promoting material.

In the present invention, the compound having the partial structure represented by formula (1) above is used as the hole transport promoting material.

A preferred embodiment of the organic electronic element of the present invention is a photoelectric conversion element. The photoelectric conversion element comprises a first electrode, a second electrode and an organic layer disposed between the first electrode and the second electrode.

The element configuration of the photoelectric conversion element, serving as an example of the organic electronic element, will be described below.

### <Configuration of Photoelectric Conversion Element>

The photoelectric conversion element according to the present invention comprises a first electrode, a second electrode and an organic layer disposed between the first electrode and the second electrode, and the organic layer includes a hole transport region. The hole transport region refers to a region between the first electrode and the light-receiving layer and includes, for example, a hole transport layer and a hole transport promoting layer.

In the present invention, the compound having the partial structure represented by formula (1) above can be used as a hole transport promoting material contained in the hole transport promoting layer.

The hole transport region is preferably adjacent to the first electrode.

The photoelectric conversion element can include other layers. Examples of other layers include layers commonly used in photoelectric conversion elements. Examples of such layers include, but not limited to, light-receiving layers, electron transport layers, hole blocking layers, electron blocking layers and buffer layers.

For example, in the photoelectric conversion element according to the present invention, the first electrode, the hole transport promoting layer, the hole transport layer and the second electrode are stacked on top of each other in this order, or the first electrode, a layer comprising a mixture of the hole transport material forming the hole transport layer and the compound having the partial structure represented by formula (1) above and the second electrode are stacked on top of each other in this order.

In addition, for example, in the photoelectric conversion element, the first electrode, the hole transport promoting layer and the hole transport layer may be stacked adjacent to each other in this order, or another layer such as a buffer layer may be disposed between the first electrode and the hole transport promoting layer or between the hole transport promoting layer and the hole transport layer.

In one embodiment, in the photoelectric conversion element according to the present invention, the first electrode, the hole transport promoting layer, the hole transport layer, the light-receiving layer and the second electrode are stacked on top of each other in this order. In another embodiment, in the photoelectric conversion element according to the present invention, the first electrode, the hole transport promoting layer, the hole transport layer, the light-receiving layer, the electron transport layer and the second electrode are stacked on top of each other in this order. These layers may be stacked adjacent to each other, or another layer may be disposed between any two layers.

The photoelectric conversion element may receive light either on the first electrode side or on the second electrode side, and either the first electrode or the second electrode may be a transparent electrode. For example, the photoelectric conversion element may have a structure in which a transparent electrode (second electrode), the electron transport layer, the light-receiving layer, the hole transport layer, the hole transport promoting layer and a metal electrode (first electrode) are stacked on top of each other in this order. Alternatively, the photoelectric conversion element may have a structure in which a transparent electrode (first electrode), the hole transport promoting layer, the hole transport layer, the light-receiving layer, the electron transport layer and a metal electrode (second electrode) are stacked on top of each other in this order. Furthermore, both the first electrode and the second electrode may be transparent electrodes.

Next, the compound having the partial structure represented by formula (1) and contained as the hole transport promoting material in the organic layer of the organic electronic element of the present invention will be described.

### <Compound Having Partial Structure Represented by Formula (1)>

The organic layer of the organic electronic element of the present invention contains the compound having the partial structure represented by the following formula (1).

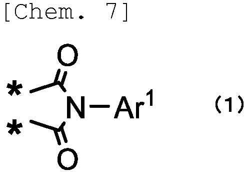

In formula (1),
in formula (1), * represents a bond, and formula (1) forms a cyclic imide structure;
Ar¹ represents a monocyclic or fused-ring aromatic hydrocarbon group optionally substituted with a substituent or a monocyclic or fused-ring heteroaromatic group optionally substituted with a substituent, the aromatic hydrocarbon group may be a group in which a plurality of aromatic hydrocarbon groups are linked together directly or with a linking group therebetween, the heteroaromatic group may be a group in which a plurality of heteroaromatic groups are linked together directly or with a linking group therebetween, and Ar¹ may be a group in which the aromatic hydrocarbon group and the heteroaromatic group are linked together;
the substituent with which the aromatic hydrocarbon group or the heteroaromatic group is optionally substituted is a cyano group, a fluoro group, a chloro group, a bromo group, an iodo group, a trifluoromethyl group, a methyl group, a fluoroalkyl group having 2 to 10 carbon atoms, a fluoroalkoxy group having 1 to 10 carbon atoms or an alkyl group having 2 to 10 carbon atoms.

**"May** be a group in which the aromatic hydrocarbon group and the heteroaromatic group are linked together" means, for example, that the group may be a group in which a monocyclic or fused-ring aromatic hydrocarbon group or aromatic hydrocarbon groups linked together are linked to a monocyclic or fused-ring heteroaromatic group or heteroaromatic groups linked together directly or with a linking group therebetween.

In formula (1) above, the monocyclic or fused-ring aromatic hydrocarbon group is preferably an aromatic hydrocarbon group having 6 to 30 carbon atoms. In addition, the monocyclic or fused-ring heteroaromatic group is preferably a heteroaromatic group having 3 to 20 carbon atoms.

Ar¹ is preferably a phenyl group, a naphthyl group, a pyridyl group, a pyrimidyl group, a pyrazyl group, a quinolyl group, a quinoxalyl group, a quinazolyl group or a triazyl group, each optionally substituted.

Examples of substituents with which Ar¹ is optionally substituted include a cyano group, a fluoro group, a chloro group, a bromo group, an iodo group, a trifluoromethyl group, a methyl group, a fluoroalkyl group having 2 to 10 carbon atoms, a fluoroalkoxy group having 1 to 10 carbon atoms or an alkyl group having 2 to 10 carbon atoms.

Specific examples of Ar¹ include, but not particularly limited to, a phenyl group, a naphthyl group, an anthryl group, a phenanthryl group, a pyrenyl group, a fluoranthenyl group, a triphenylenyl group, a biphenyl group, a terphenyl group, a methylphenyl group, a methylanthryl group, a methylphenanthryl group, a fluorophenyl group, a fluoronaphthyl group, a fluoroanthryl group, a fluorophenanthryl group, a cyanophenyl group, a cyanonaphthyl group, a cyanoanthryl group, a cyanophenanthryl group, a pyridylphenyl group, a pyridylnaphthyl group, a pyridylanthryl group, a pyridylphenanthryl group, a dimethylphenyl group, a dicyanophenyl group, a bis(trifluoromethyl)phenyl group, a bis(trifluoromethyl)naphthyl group, a bis(trifluoromethyl)anthryl group, a bis(trifluoromethyl)phenanthryl group, a phenyl group substituted with a cyano group and a fluoro group, a phenyl group substituted with a cyano group and a trifluoromethyl group, a phenyl group substituted with a fluoro group and a cyano group, a phenyl group substituted with a fluoro group and a trifluoromethyl group, a trifluorophenyl group, a pentafluorophenyl group, a bispyridylphenyl group, a bipyridyl group, a terpyridyl group, a quinolyl group, an isoquinolyl group, a pyrazyl group, a pyrimidyl group, a quinoxalyl group, a quinazolyl group, a methylpyridyl group, a methylquinolyl group, a methylisoquinolyl group, a methylpyrazyl group, a methylpyrimidyl group, a dimethylpyridyl group, a dimethylquinolyl group, a dimethylisoquinolyl group, a dimethylpyrazyl group, a dimethylpyrimidyl group, a pyridyl group substituted with a methyl group and a cyano group, a fluoropyridyl group, a fluoroquinolyl group, a fluoroisoquinolyl group, a fluoropyrazyl group, a fluoropyrimidyl group, a fluoroquinoxalyl group, a fluoroquinazolyl group, a difluoropyridyl group, a difluoroquinolyl group, a difluoroisoquinolyl group, a difluoropyrazyl group, a difluoropyrimidyl group, a difluoroquinoxalyl group, a difluoroquinazolyl group, a trifluoropyridyl group, a trifluoroquinolyl group, a trifluoroisoquinolyl group, a trifluoropyrazyl group, a trifluoropyrimidyl group, a trifluoroquinoxalyl group, a trifluoroquinazolyl group, a tetrafluoropyridyl group, a tetrafluoroquinolyl group, a tetrafluoroisoquinolyl group, a trifluoromethylpyridyl group, a trifluoromethylquinolyl group, a trifluoromethylisoquinolyl group, a trifluoromethylpyrazyl group, a trifluoromethylpyrimidyl group, a trifluoromethylquinoxalyl group, a trifluoromethylquinazolyl group, a bistrifluoromethylpyridyl group, a bistrifluoromethylquinolyl group, a bistrifluoromethylisoquinolyl group, a bistrifluoromethylpyrazyl group, a bistrifluoromethylpyrimidyl group, a bistrifluoromethylquinoxalyl group, a bistrifluoromethylquinazolyl group, a tris(trifluoromethyl)pyridyl group, a tris(trifluoromethyl)quinolyl group, a tris(trifluoromethyl)isoquinolyl group, a tris(trifluoromethyl)pyrazyl group, a tris(trifluoromethyl)pyrimidyl group, a tris(trifluoromethyl)quinoxalyl group, a tris(trifluoromethyl)quinazolyl group, a cyanopyridyl group, a cyanoquinolyl group, a cyanoisoquinolyl group, a cyanopyrazyl group, a cyanopyrimidyl group, a cyanoquinoxalyl group, a cyanoquinazolyl group, a dicyanopyridyl group, a dicyanoquinolyl group, a dicyanoisoquinolyl group, a dicyanopyrazyl group, a dicyanopyrimidyl group, a dicyanoquinoxalyl group, a dicyanoquinazolyl group, a tricyanopyridyl group, a tricyanoquinolyl group, a tricyanoisoquinolyl group, a tricyanopyrazyl group, a tricyanopyrimidyl group, a tricyanoquinoxalyl group, a tricyanoquinazolyl group, a pyridyl group substituted with a cyano group and a fluoro group, a pyridyl group substituted with a cyano group and a trifluoromethyl group, a pyridyl group substituted with a fluoro group and a cyano group, a pyridyl group substituted with a fluoro group and a trifluoromethyl group, a quinoxalyl group substituted with a cyano group and a trifluoromethyl group, a quinazolyl group substituted with a cyano group and a trifluoromethyl group, a triazyl group, a diphenyltriazyl group, a dicyanotriazyl group, a dipyridyltriazyl group, a bis(trifluoromethyl)triazyl group, a bisadamantyltriazyl group, a bisadamantylpyrimidyl group, a bisadamantylpyridyl group, a biscyclohexyltriazyl group, a biscyclohexylpyrimidyl group and a biscyclohexylpyridyl group. Ar¹ may also be a group in which two or more of these groups are combined together.

The compound having the partial structure represented by formula (1) above is preferably substituted with an electron-withdrawing substituent since the LUMO level of the hole transport promoting material can be made deeper and closer to the HOMO level of the hole transport material. In formula (1) above, Ar¹ is preferably an electron-withdrawing substituent.

Here, the electron-withdrawing substituent refers to a substituent with which the LUMO level of the hole transport promoting material can be made deeper than without the substituent.

The electron-withdrawing substituent is not particularly limited and can be selected as appropriate. Of the groups used as Ar¹ above, the electron-withdrawing substituent may **be,** for example:
a cyano group, a fluoro group, a chloro group, a bromo group, an iodo group, a trifluoromethyl group, a fluoroalkyl group having 2 to 10 carbon atoms or a fluoroalkoxy group having 1 to 10 carbon atoms;
an aromatic hydrocarbon group substituted with one or more of a cyano group, a fluoro group, a chloro group, a bromo group, an iodo group and a trifluoromethyl group;
a heteroaromatic group substituted with one or more of a cyano group, a fluoro group, a chloro group, a bromo group, an iodo group and a trifluoromethyl group; or
a group in which two or more selected from the group consisting of the above groups are combined together.

The Hammet constant is known as a measure of electron-withdrawing ability. Preferred examples of electron-withdrawing substituents also include substituents that exhibit positive Hammet constants.

The aromatic hydrocarbon group or the heteroaromatic group for Ar¹ in formula (1) above may be substituted with a plurality of substituents. When the aromatic hydrocarbon group or the heteroaromatic group is substituted with a plurality of substituents, those substituents may be the same as or different from each other.

In formula (1) above, Ar¹ is preferably a phenyl group, a pyridyl group, a pyrazyl group or a pyrimidyl group, each optionally substituted with at least one group selected from the group consisting of a cyano group, a fluoro group and a trifluoromethyl group.

The compound having the partial structure represented by formula (1) above is preferably a compound represented by the following formula (2).

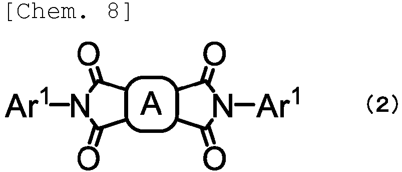

In formula (2), Ar¹ represents the same group as Ar¹ in formula (1) above. One Ar¹ group is the same group as the other Ar¹ group.

In formula (2), the ring A represents a monocyclic or fused-ring aromatic hydrocarbon ring. The aromatic hydrocarbon ring may be a ring in which a plurality of aromatic hydrocarbon rings are linked together directly or with a linking group therebetween.

In formula (2), the ring A is preferably, for example, a ring represented by any of the following (A-1) to (A-9).

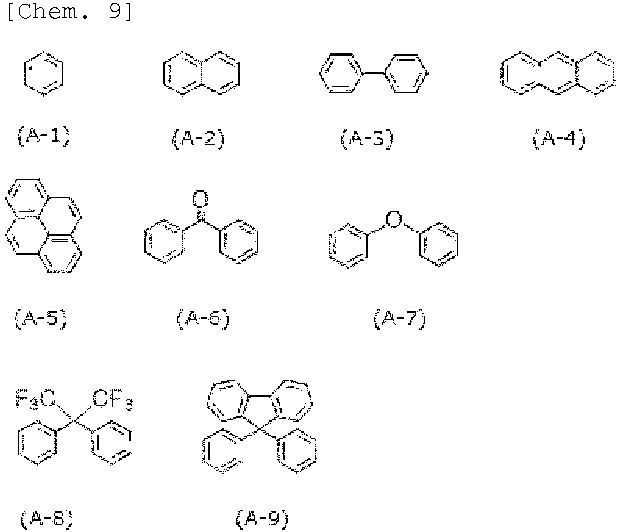

In formula (2), particularly, the ring A is more preferably formula (A-1) or formula (A-2) above.

In formula (2), when the ring A is formula (A-1) above, the imide compound of the present invention is represented by, for example, the following formula (2-1). When the ring A is formula (A-2) above, the imide compound of the present invention is represented by, for example, the following formula (2-2) or the following formula (2-3).

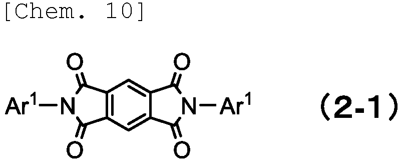

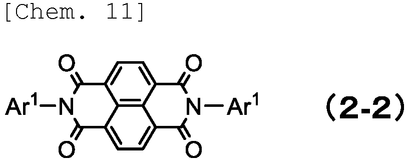

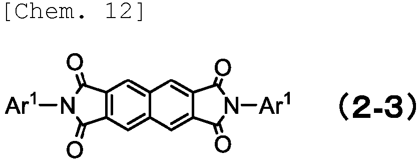

Preferred specific examples of compounds having the partial structure represented by formula (1) (more specifically, compounds represented by formula (2)) are given below, although the compound of the present invention is not limited thereto.

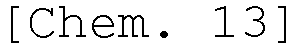

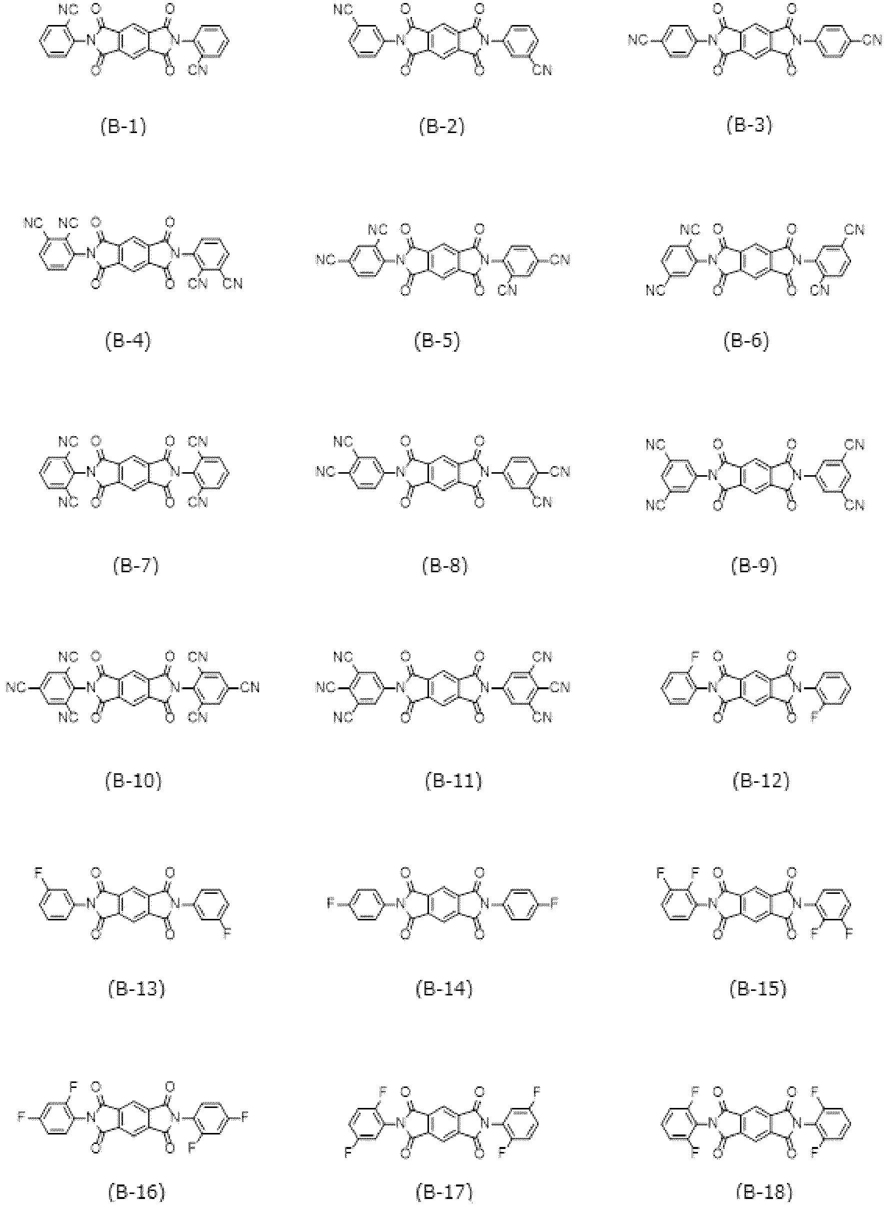

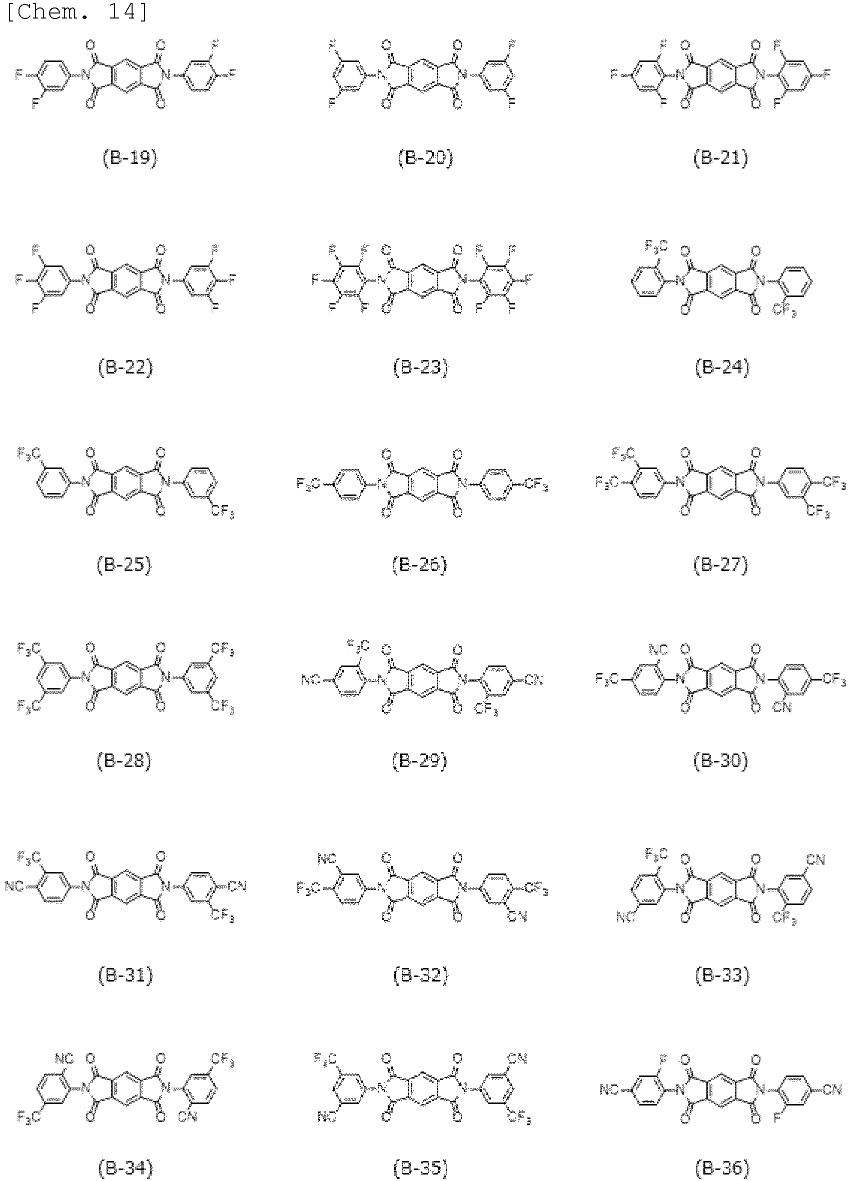

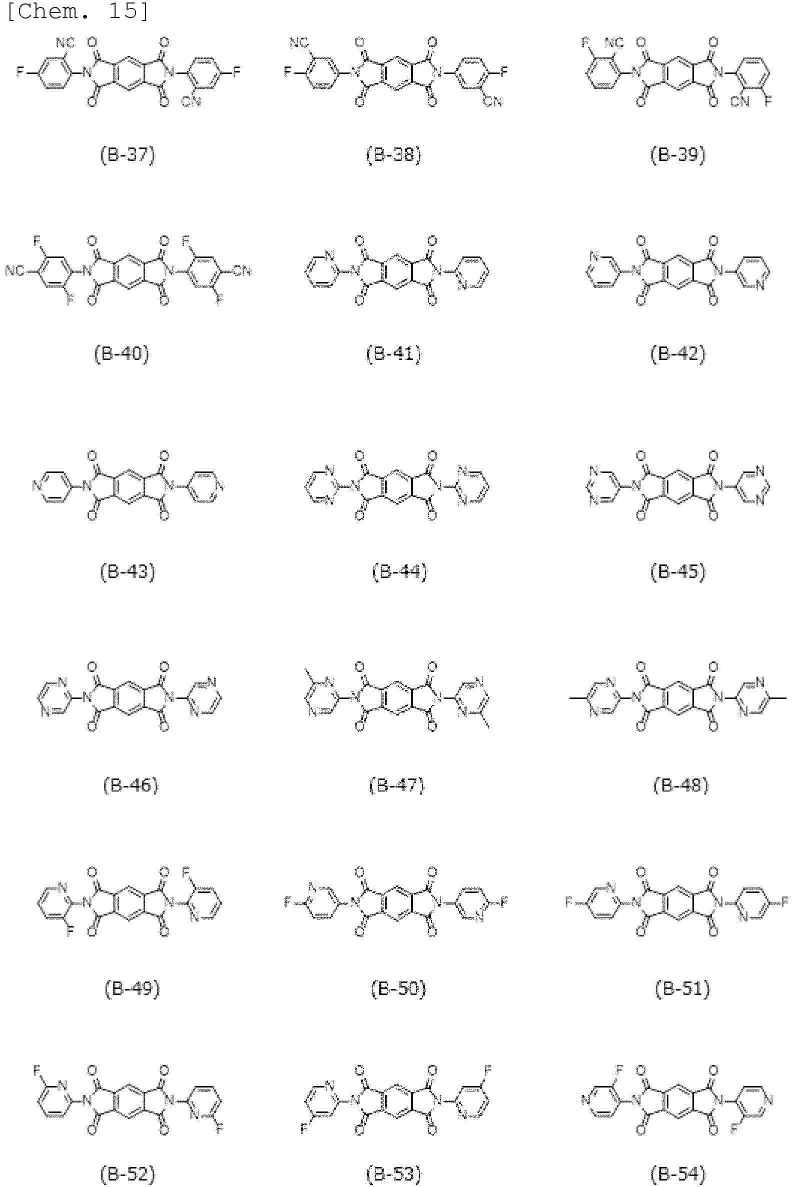

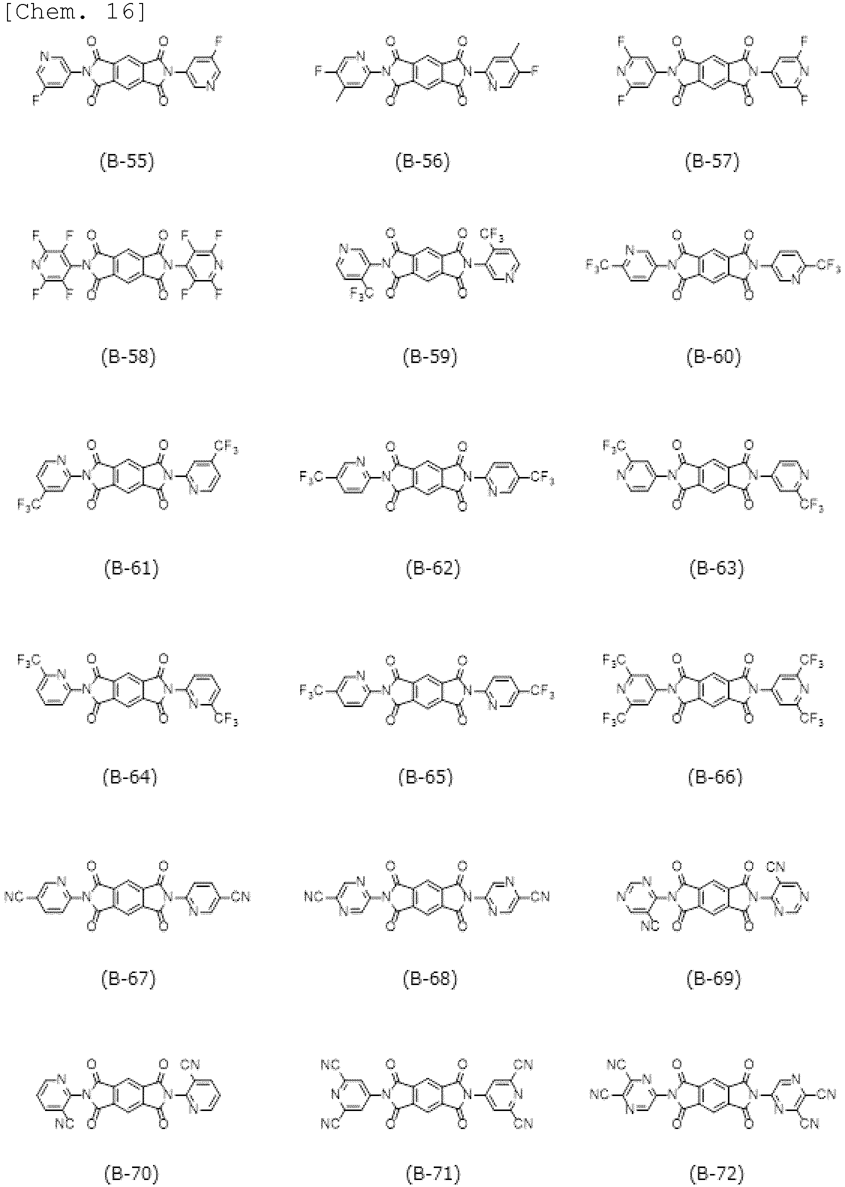

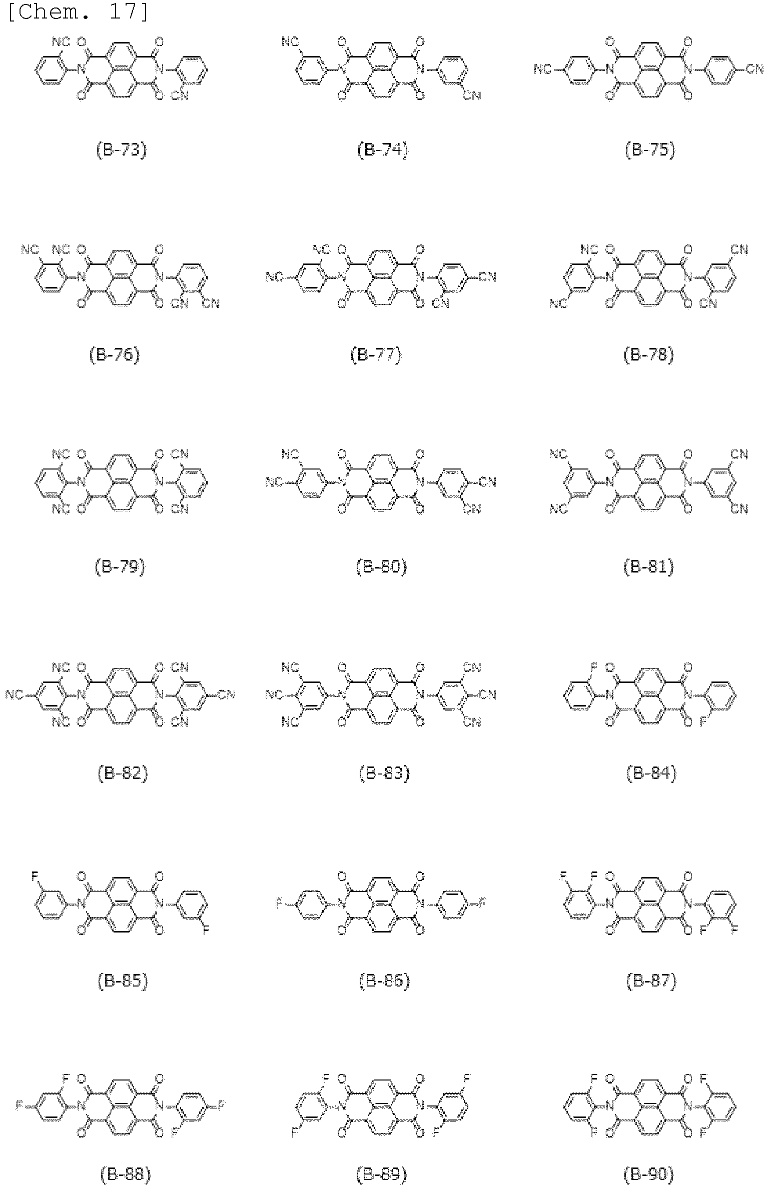

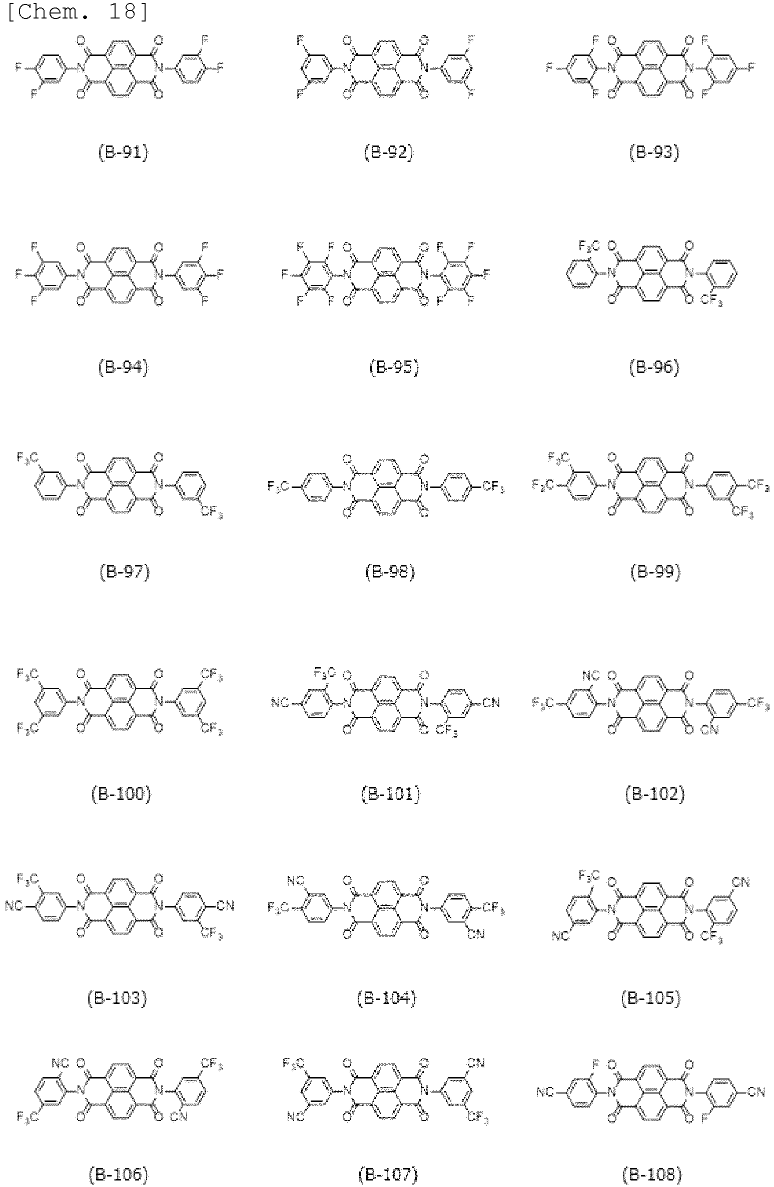

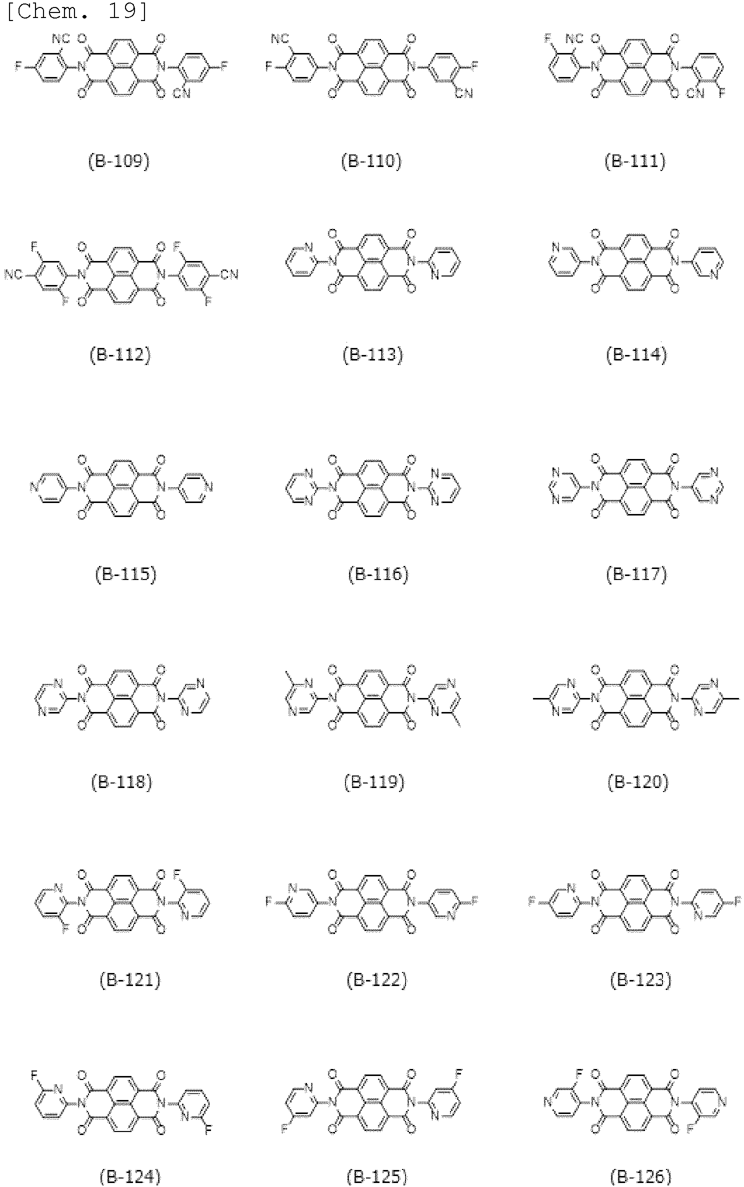

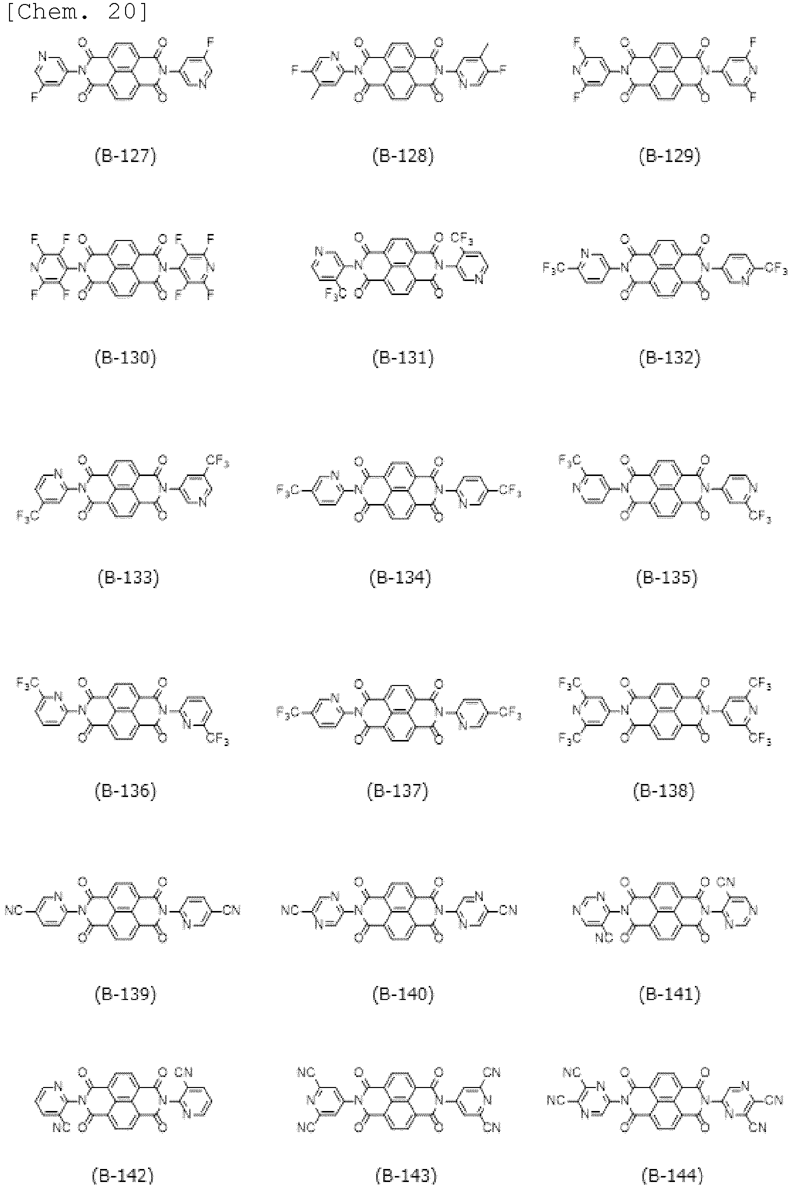

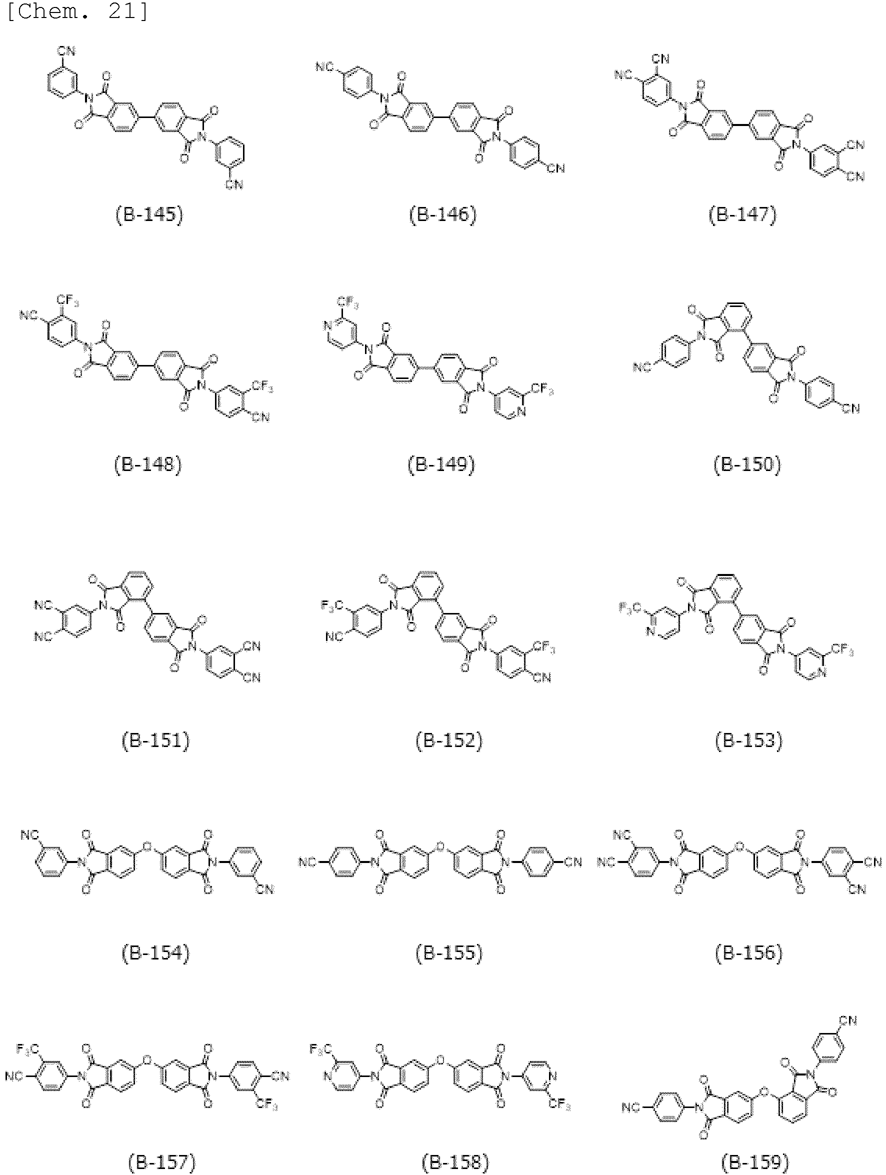

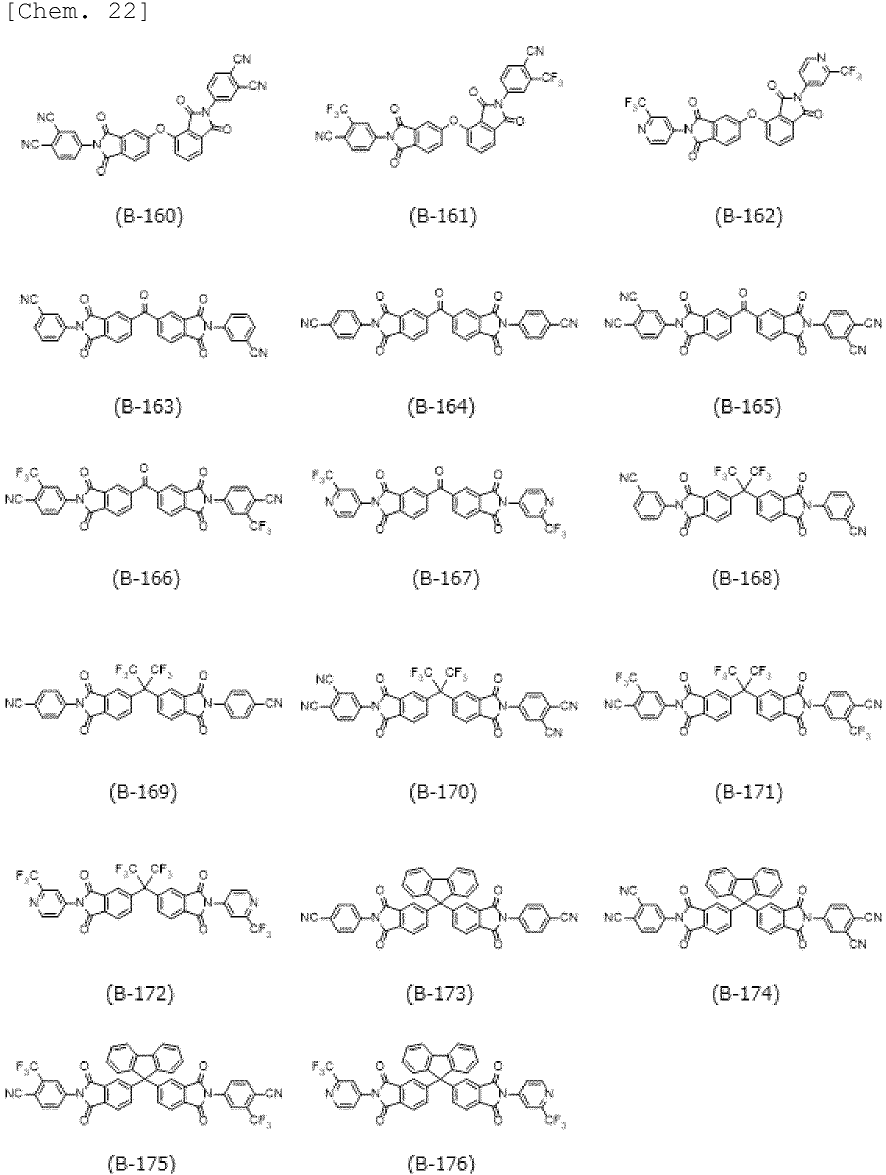

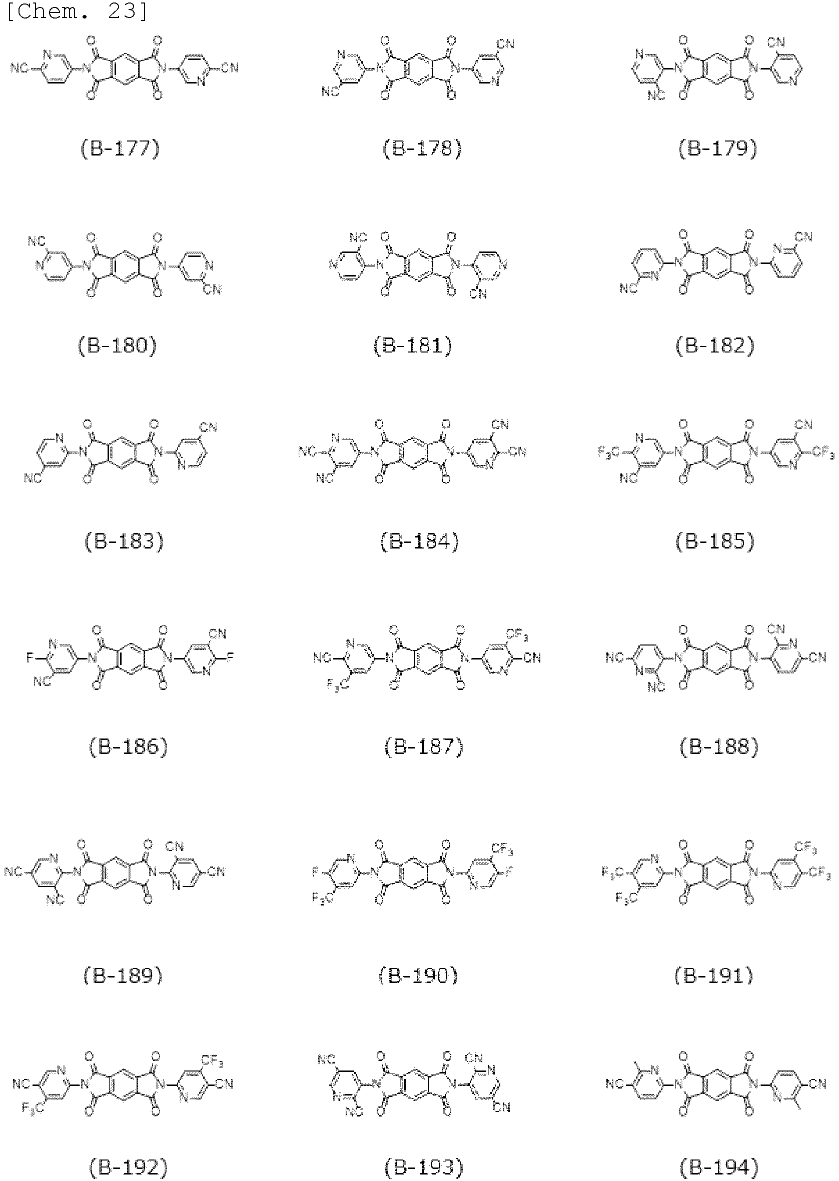

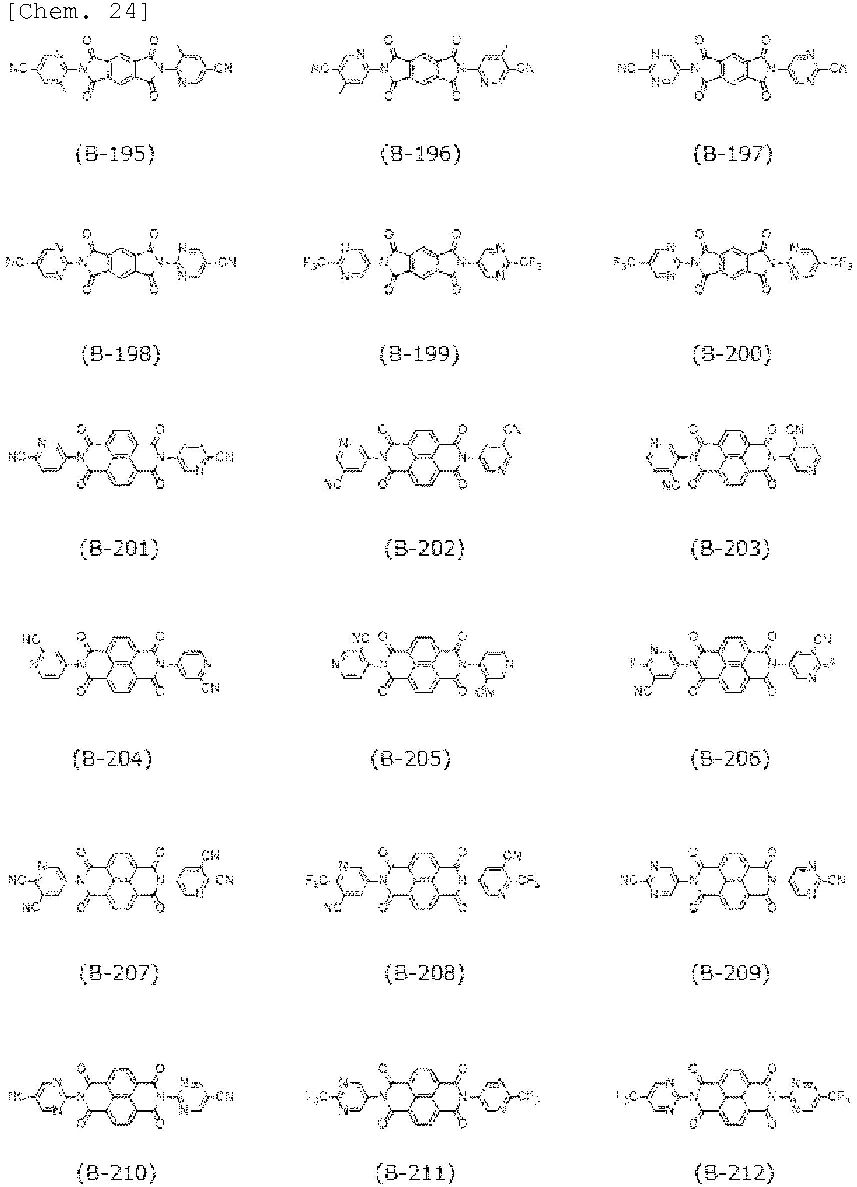

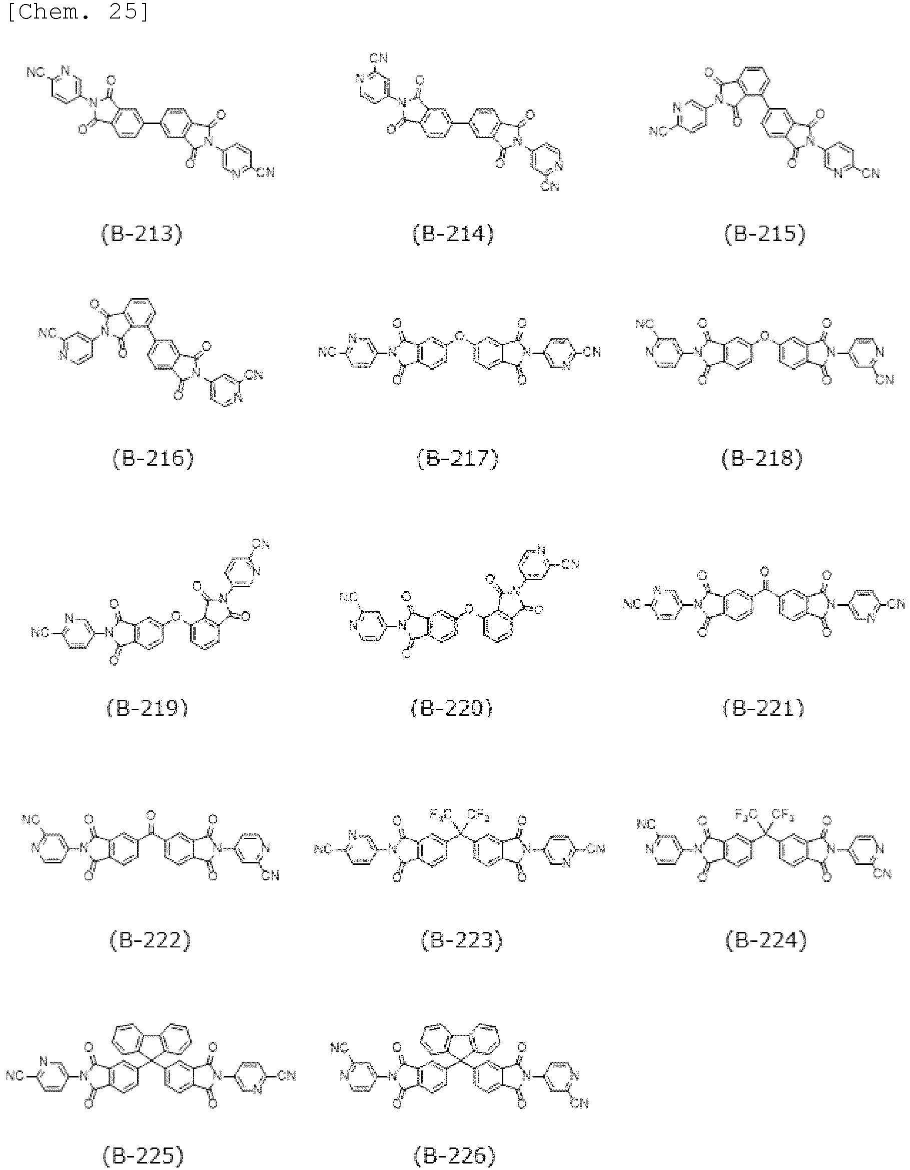

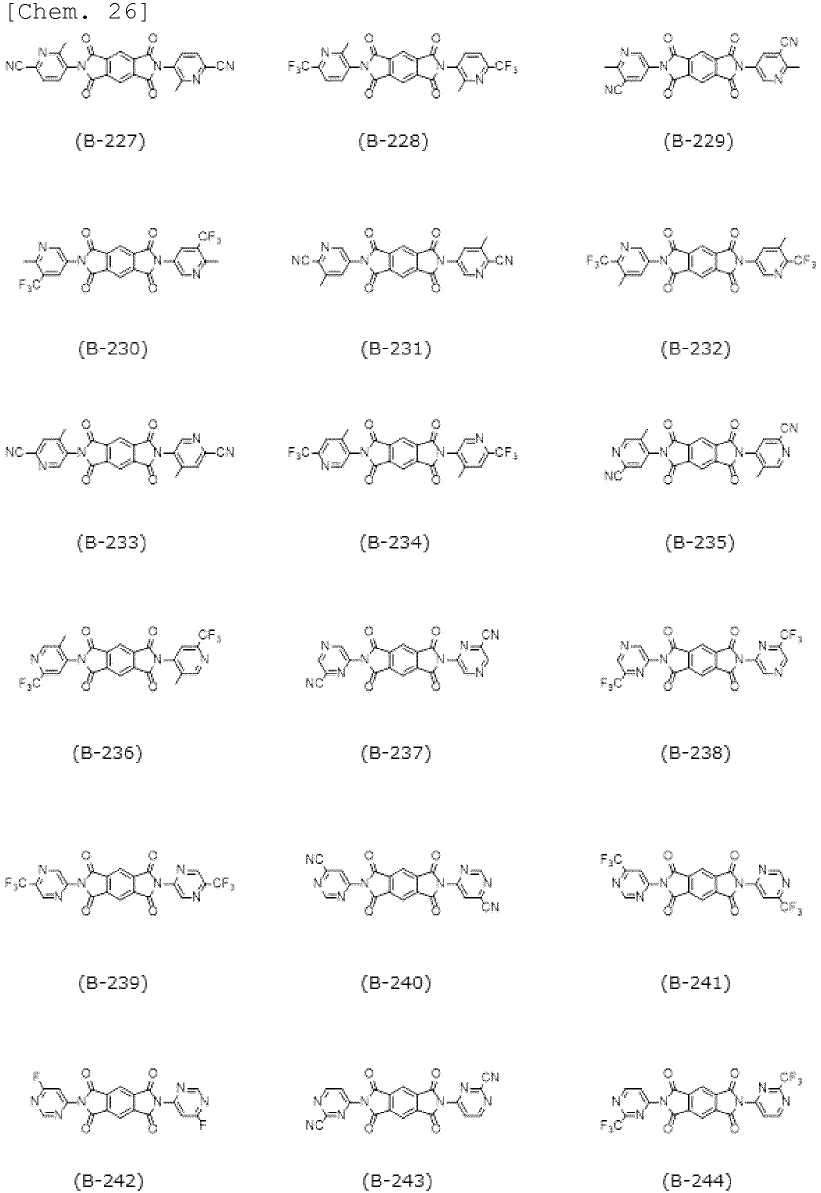

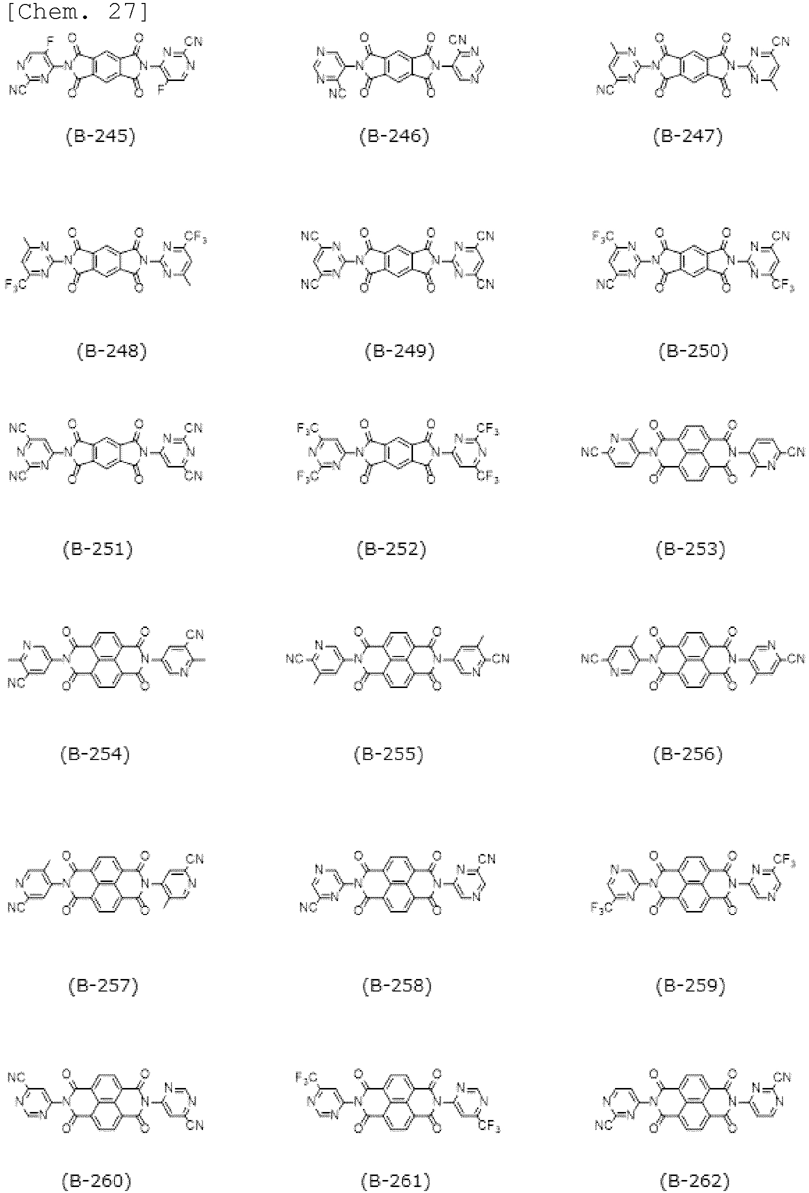

The compound represented by formula (2) above can be synthesized by known methods or combinations thereof. For example, the compound represented by formula (2) can be synthesized according to (Journal of Materials Chemistry A, 2015, 3, 878-885). 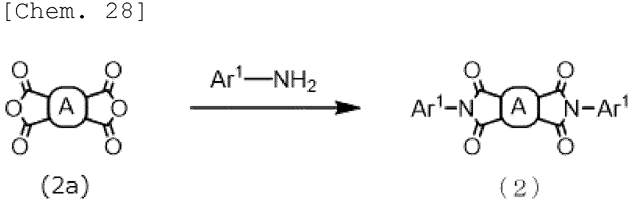 (wherein
Ar¹ represents the same group as Ar¹ in formula (1) above, and one Ar¹ group is the same group as the other Ar¹ group; and
the ring A represents a monocyclic or fused-ring aromatic hydrocarbon ring, and the aromatic hydrocarbon ring may be a ring in which a plurality of aromatic hydrocarbon rings are linked together directly or with a linking group therebetween).

### <<Operation and Effects of Compound Represented by Formula (2)>>

The compound represented by formula (2) has an imide skeleton as a partial structure. This strong acceptor skeleton is expected to produce strong interaction with the HOMO orbital of the hole transport material. That is, when a layer (e.g., the hole transport promoting layer) in the organic electronic element (e.g., a photoelectric conversion element) contains the compound represented by formula (2), the compound represented by formula (2) is expected to enhance the interaction with the HOMO orbital of the adjacent hole transport layer and promote carrier transfer between the hole transport layer and the hole transport promoting layer. Thus, the compound represented by formula (2) has an extremely deep LUMO level and is therefore expected to allow smooth transfer of holes between the hole transport layer and the electrode.

In addition, the compound represented by formula (2) has a symmetrical imide structure and can therefore be expected to provide, for example, thermal stability and high reduction resistance.

As described above, the inventors have found that the compound represented by formula (2) can be effectively used as a hole transport promoting material that allows smooth transfer of holes between the hole transport layer and the electrode. The inventors have actually confirmed that the use of a hole transport material in combination with the compound represented by formula (2) (hole transport promoting material) promotes the hole transport ability of a photoelectric conversion element. Specifically, the inventors have confirmed that the use of the compound represented by formula (2), which is the hole transport promoting material of the present application, in a photoelectric conversion element can reduce the energy barrier against transfer of carriers generated in the light-receiving layer to the electrode side.

### <Embodiments>

The multilayer configuration of the organic electronic element (e.g., a photoelectric conversion element) of the present invention may be, for example, the following configuration (i) or (ii):
(i): first electrode/hole transport promoting layer/hole transport layer/light-receiving layer/second electrode
(ii): first electrode/hole transport promoting layer/hole transport layer/light-receiving layer/electron transport layer/second electrode

The photoelectric conversion element according to the present invention will be more specifically described below with reference to Fig. 1 by taking the configuration (ii) as an example. Fig. 1 is a schematic sectional view illustrating an example of the multilayer configuration of the photoelectric conversion element according to the present invention.

### <<First Embodiment>>

A photoelectric conversion element according to a first embodiment is an organic image capture element or an optical sensor having the multilayer configuration illustrated in Fig. 1.

A photoelectric conversion element 1 includes, in order, a first electrode 11 (first electrode), a hole transport promoting layer 12, a hole transport layer 13, a light-receiving layer 14, an electron transport layer 15 and a second electrode 16 (second electrode). It is noted that some of these layers may be omitted, or conversely, other layers may be added.

In the photoelectric conversion element 1 illustrated in Fig. 1, light incident from above the first electrode 11, which is transparent, is received by the light-receiving layer 14. In Fig. 1, light is illustrated as being incident on the side surface of the light-receiving layer 14 for illustration purposes. A voltage is applied to the photoelectric conversion element 1 such that, of electric charges (holes and electrons) generated by photoelectric conversion in the light-receiving layer 14, holes are transferred to the first electrode 11 and electrons are transferred to the second electrode 16. That is, the first electrode 11 serves as a hole collection electrode, whereas the second electrode 16 serves as an electron collection electrode. In Fig. 1, a substrate disposed on the top surface of the first electrode 11 is omitted. The substrate herein is not particularly limited, and examples thereof include glass substrates, quartz substrates and plastic substrates. In addition, in a configuration in which light is incident on the substrate side, the substrate is transparent to the wavelength of the light. The individual layers will be described below.

### [First Electrode 11]

The first electrode 11 or the second electrode 16 is disposed on the substrate.

For a photoelectric conversion element having a configuration in which light enters the light-receiving layer 14 through the first electrode 11, the first electrode is formed of a transparent material that transmits the light or essentially transmits the light. Here, "transmit light" refers to an average transmittance of 80% or more, and "essentially transmit light" refers to an average transmittance of 50% or more. That is, in the present specification, "transparent" refers to an average transmittance of 50% or more.

Examples of transparent materials used for the first electrode 11 or the second electrode 16 include, but not particularly limited to, indium tin oxide (ITO), indium zinc oxide (IZO), tin oxide, aluminum-doped tin oxide, magnesium indium oxide, nickel tungsten oxide, other metal oxides, metal nitrides such as gallium nitride, metal selenides such as zinc selenide and metal sulfides such as zinc sulfide.

For a photoelectric conversion element having a configuration in which light enters the light-receiving layer 14 only through the second electrode 16 side, the transmission characteristics of the first electrode 11 are not important. Therefore, examples of materials used for the first electrode in this case include gold, iridium, molybdenum, palladium and platinum.

### [Hole Transport Promoting Layer 12]

The hole transport promoting layer 12 is disposed between the first electrode 11 and the hole transport layer 13 described later. The hole transport promoting layer 12 is provided to promote hole transport from the hole transport layer 13 to the first electrode 11. The hole transport promoting layer 12 contains the compound having the partial structure represented by formula (1) described above. The hole transport promoting layer 12 may simultaneously contain a compound other than the compound having the partial structure represented by formula (1). Examples of compounds that can be contained in the hole transport promoting layer 12 include conventionally known hole transport materials, for example, compounds used for the hole transport layer 13 described later.

### [Hole Transport Layer 13]

The hole transport layer 13 is disposed between the hole transport promoting layer 12 and the light-receiving layer 14.

The hole transport layer 13 functions to transport holes generated in the light-receiving layer 14 from the light-receiving layer 14 to the first electrode 11 and to block movement of electrons generated in the light-receiving layer 14 to the first electrode 11 side. The hole transport layer 13 may also function to block electron injection from the first electrode 11, depending on the use.

The hole transport layer 13 may have a single-layer structure composed of one or more materials or a multilayer structure composed of a plurality of layers having the same composition or different compositions. The hole transport material that can be contained in the hole transport layer 13 may be a known hole transport material. Examples of known hole transport materials include aromatic tertiary amine compounds, naphthalene compounds, anthracene compounds, tetracene compounds, pentacene compounds, phenanthrene compounds, pyrene compounds, perylene compounds, fluorene compounds, carbazole compounds, indole compounds, pyrrole compounds, picene compounds, thiophene compounds, benzotrifuran compounds, benzotrithiophene compounds, naphthodithiophene compounds, naphthothienothiophene compounds, benzodifuran compounds, benzodithiophene compounds, benzothiophene compounds, naphthobisbenzothiophene compounds, chrysenodithiophene compounds, benzothienobenzothiophene compounds and indolocarbazole compounds.

Of these, fluorene compounds, carbazole compounds, naphthodithiophene compounds, naphthothienothiophene compounds, benzodifuran compounds, benzothiophene compounds, naphthobisbenzothiophene compounds, chrysenodithiophene compounds, benzothienobenzothiophene compounds, indolocarbazole compounds and the like are preferred, and fluorene compounds, carbazole compounds, chrysenodithiophene compounds, benzothienobenzothiophene compounds and indolocarbazole compounds are particularly preferred.

### [Light-Receiving Layer 14]

The light-receiving layer 14 is disposed between the hole transport layer 13 and the electron transport layer 15 described later.

An example of a material for the light-receiving layer 14 is a material having a photoelectric conversion function.

The light-receiving layer 14 may have a single-layer structure composed of one or more materials or a multilayer structure composed of a plurality of layers having the same composition or different compositions.

Of these, it is preferred that the light-receiving layer be composed of a layer containing at least two materials (organic components) to improve the photoelectric conversion efficiency.

Examples of materials used for a light-receiving layer 14 having a single-layer structure composed of one material include (i) coumarin and derivatives thereof, quinacridone and derivatives thereof and phthalocyanine and derivatives thereof.

Examples of materials used for a light-receiving layer 14 having a single-layer structure composed of two materials include combinations of (i) coumarin and derivatives thereof, quinacridone and derivatives thereof and phthalocyanine and derivatives thereof as described above and (ii) fullerene and derivatives thereof and other acceptor materials. A light-receiving layer 4 composed of such materials may be formed by vapor deposition with a mixture of powders prepared in advance or by co-deposition in any ratio.

Examples of materials used for a light-receiving layer 14 having a single-layer structure composed of three materials include combinations of (i) coumarin and derivatives thereof, quinacridone and derivatives thereof and phthalocyanine and derivatives thereof as described above, (ii) fullerene and derivatives thereof and other acceptor materials and (iii) hole transport materials. A light-receiving layer 4 composed of such materials may be formed by vapor deposition with a mixture of powders prepared in advance or by co-deposition in any ratio.

Specific examples of (i) coumarin derivatives include coumarin 6 and coumarin 30. Specific examples of quinacridone derivatives include N,N-dimethylquinacridone. Specific examples of phthalocyanine derivatives include boron subphthalocyanine chloride and boron subnaphthalocyanine chloride (SubNC).

Specific examples of (ii) fullerene and derivatives thereof include [60]fullerene, [70]fullerene and [6,6]-phenyl-C61-butyric acid methyl ester ([60]PCBM).

Preferred compounds used as (iii) hole transport materials and specific examples thereof include the same compounds as those used for the hole transport layer 13 described above.

In addition, the material having a photoelectric conversion function is not limited to being contained only in the light-receiving layer. For example, the material having a photoelectric conversion function may also be contained in a layer (hole transport layer 13 or electron transport layer 15) adjacent to the light-receiving layer 14.

### [Electron Transport Layer 15]

The electron transport layer 15 is disposed between the light-receiving layer 14 and the second electrode 16 described later.

The electron transport layer 15 functions to transport electrons generated in the light-receiving layer 14 to the second electrode 16 and to block movement of holes from the second electrode 16, to which electrons are transported, to the light-receiving layer 14. The electron transport layer 15 may also function to block hole injection from the second electrode 16, depending on the use.

In addition, the electron transport material that can be contained in the electron transport layer 15 may be a known electron transport material. Examples of electron transport materials include fullerene, fullerene derivatives, triazine derivatives, bis(8-hydroxyquinolinato)manganese, tris(8-hydroxyquinolinato)aluminum, tris(2-methyl-8-hydroxyquinolinato)aluminum, 2,9-dimethyl-4,7-diphenyl-1,10-phenanthroline (BCP), 4,7-diphenyl-1,10-phenanthroline (Bphen), bis(2-methyl-8-quinolinolato)-4-(phenylphenolato)aluminum (BAlq), 4,6-bis(3,5-di(pyridin-4-yl)phenyl)-2-methylpyrimidine, N,N'-diphenyl-1,4,5,8-naphthalenetetracarboxylic acid diimide and N,N'-di(4-pyridyl)-1,4,5,8-naphthalenetetracarboxylic acid diimide.

The electron transport layer 15 may have a single-layer structure composed of one or more materials or a multilayer structure composed of a plurality of layers having the same composition or different compositions.

### [Second Electrode 16]

The second electrode 16 is disposed on the electron transport layer 15.

Examples of materials for the second electrode 16 include indium tin oxide (ITO), indium zinc oxide (IZO), sodium, sodium-potassium alloys, magnesium, lithium, magnesium/copper mixtures, magnesium/silver mixtures, magnesium/aluminum mixtures, magnesium/indium mixtures, aluminum/aluminum oxide (Al₂O₃) mixtures, indium, lithium/aluminum mixtures, gold, platinum, rare earth metals and molybdenum oxide. The first electrode 11 and the second electrode 16 may be the same as or different from each other.

### [Method for Forming Each Layer]

The layers other than the first electrode 11 and the second electrode 16 described above can be formed, for example, by forming a thin film from the materials for the individual layers (optionally together with a material such as a binder resin and a solvent) by a known method such as vacuum deposition, spin coating, casting or the Langmuir-Blodgett (LB) method.

The thicknesses of the thus-formed layers are not particularly limited and can be selected as appropriate depending on the situation, typically in the range of 5 nm or more and 5 µm or less.

The first electrode 11 and the second electrode 16 can be formed by forming a thin film from an electrode material by a method such as vapor deposition or sputtering. During vapor deposition or sputtering, a pattern may be formed through a mask having the desired shape. Alternatively, after a thin film is formed by a method such as vapor deposition or sputtering, a pattern having the desired may be formed by photolithography.

The first electrode 11 and the second electrode 16 preferably have a thickness of 1 µm or less, more preferably 10 nm or more and 200 nm or less.

The materials forming the first electrode 11 and the second electrode 16 may be exchanged as needed (also known as an inverted structure). This structure results in a photoelectric conversion element having a configuration in which light enters the light-receiving layer 14 through the second electrode 16

An image capture element including the photoelectric conversion element of this embodiment is applicable to, for example, an image capture element for a digital camera or a digital video camera and an image capture element built into a cellular phone or the like. An optical sensor is applicable to, for example, television remote controllers, air conditioner switches and opening and closing of automatic doors.

### <<Second Embodiment>>

A photoelectric conversion element according to a second embodiment of the present invention is a solar cell having the multilayer configuration illustrated in Fig. 1.

A solar cell 1 includes a hole transport promoting layer 12 and a hole transport layer 13 disposed between a first electrode 11 and a light-receiving layer 14 and an electron transport layer 15 disposed between a second electrode 16 and the light-receiving layer 14. It is noted that some of these layers may be omitted, or conversely, other layers may be added.

### [First Electrode 11]

The first electrode 11 is made of, for example, a transparent material, and the transparent material in the first embodiment can be used as the transparent material. The first electrode 11 may be formed on any substrate (e.g., a transparent substrate such as glass, a plastic or a polymer film).

### [Hole Transport Promoting Layer 12]

The material for the hole transport promoting layer 12 is the same as the material (the compound having the partial structure represented by formula (1)) for the hole transport promoting layer 12 in the first embodiment. Apart from the material in the first embodiment, the material for the hole transport promoting layer 12 may contain conventionally known hole transport materials.

### [Hole Transport Layer 13]

The material for the hole transport layer 13 is the same as the material for the hole transport layer 13 in the first embodiment. Apart from the hole transport material in the first embodiment, the material for the hole transport layer 13 may contain conventionally known hole transport materials.

### [Light-Receiving Layer 14]

The material for the light-receiving layer 14 may be any material containing an electron donor material and an electron acceptor material. The light-receiving layer 14 may be of a planar junction type, in which an electron donor material and an electron acceptor material are joined in a plane, or of a bulk heterojunction type, in which a film is formed of a mixture of an electron donor material and an electron acceptor material.

The electron donor material is preferably, but not particularly limited to, an organic semiconductor. Examples of electron donor materials include polymer compounds such as polythiophene derivatives, polyfluorene derivatives and polyphenylenevinylene derivatives and copolymers thereof, phthalocyanine derivatives and metal complexes thereof, porphyrin derivatives and metal complexes thereof, acene derivatives such as pentacene and low-molecular-weight compounds such as diamine derivatives. As the electron donor material, an inorganic semiconductor can be used in combination with the organic semiconductor as long as the inorganic semiconductor does not interfere with the advantages of the present invention.

The electron acceptor material is preferably, but not particularly limited to, an organic semiconductor. Examples of electron acceptor materials include fullerene derivatives, perylene derivatives and naphthalene derivatives.

### [Electron Transport Layer 15]

As the material for the electron transport layer 15, the electron transport material in the first embodiment can be used. As the electron transport material, alkali metal halides such as sodium fluoride and cesium fluoride, alkaline earth metal halides such as calcium fluoride, carbonates such as cesium carbonate and inorganic n-type semiconductors such as titanium oxide and zinc oxide may also be used.

### [Second Electrode 16]

Examples of materials for the second electrode 16 include, but not limited to, metals such as magnesium, calcium, sodium, potassium, titanium, indium, yttrium, lithium, gadolinium, aluminum, silver, tin and lead and alloys thereof.

The materials forming the first electrode 11 and the second electrode 16 may be exchanged as needed (also known as an inverted structure). This structure results in a photoelectric conversion element having a configuration in which light enters the light-receiving layer 14 through the second electrode 16

### [Method for Forming Each Layer]

The method for forming each layer is not particularly limited. For example, the first electrode 11, the hole transport promoting layer 12, the hole transport layer 13, the light-receiving layer 14, the electron transport layer 15 and the second electrode 16 may be sequentially formed on top of each other on the substrate by a method such as vapor deposition, spin coating, casting or pattern transfer. Alternatively, after the hole transport promoting layer 12, the hole transport layer 13, the light-receiving layer 14 and the electron transport layer 15 are formed on top of each other, the first electrode 11 and the second electrode 16 may be formed thereon by a method such as transfer, vapor deposition or sputtering.

The organic electronic element (photoelectric conversion element) of the present invention and the method for forming each layer forming the element are not limited to those shown in the embodiments described above. For example, the materials for the first electrode, the light-receiving layer, the electron transport layer and the second electrode can be replaced with other known materials as appropriate. In addition, the hole transport promoting layer and the hole transport layer can be replaced with a layer formed of a mixture of the compound having the partial structure represented by formula (1) and the hole transport material.

### (Compound That Can Be Used in Organic Electronic Element)

The present invention provides a novel compound that is used in an organic electronic element and that can improve the hole transport ability thereof.

A novel imide compound of the present invention that can be used in an organic electronic element is represented by the following formula (3). 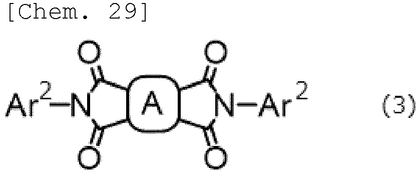 (wherein
Ar² represents a pyridyl group, a pyrazyl group or a pyrimidyl group, each substituted with at least one group selected from the group consisting of a cyano group, a fluoro group and a trifluoromethyl group, and one Ar² group is the same group as the other Ar² group, and
the ring A represents any of the following (A-1) to (A-9)).

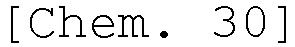

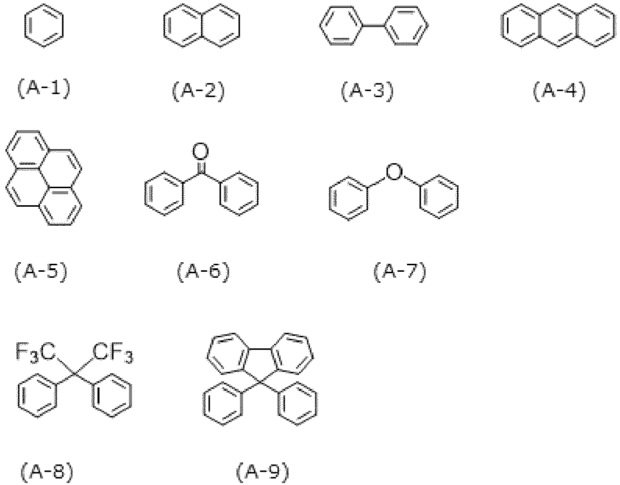

In formula (3) above, of the pyridyl group, the pyrazyl group and the pyrimidyl group, Ar² is preferably the pyridyl group from the viewpoint of physical properties.

In formula (3) above, the ring A is preferably represented by formula (A-1) or formula (A-2) above.

The imide compound wherein the ring A in formula (3) above is formula (A-1) or formula (A-2) above has the same meaning as that represented by the ring A in formula (2) above.

### EXAMPLES

The present invention will be more specifically described with reference to the examples below, although the present invention should not be construed as being limited to these examples.

### [Compounds Used for Evaluation]

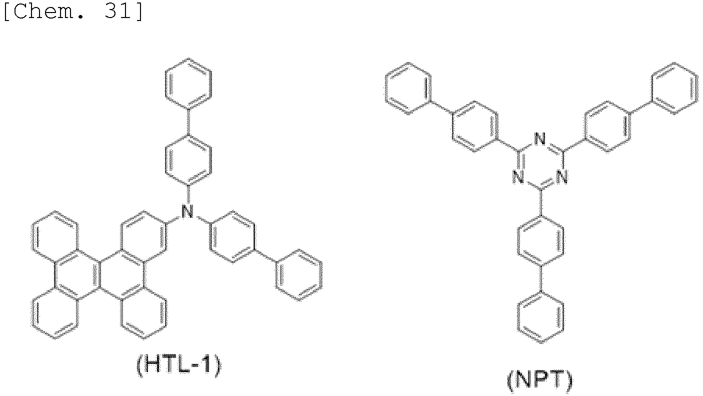

### [Synthesis Example 1: Synthesis of Compound (B-3)]

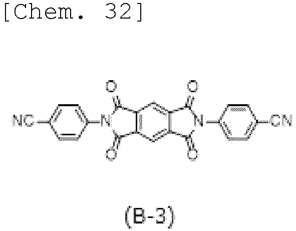

In a nitrogen stream, 1.1 g (5.0 mmol) of benzene-1,2,4,5-tetracarboxylic anhydride, 1.2 g (10.5 mmol) of 4-aminobenzonitrile, 0.6 g (4.3 mmol) of isoquinoline and 20 mL of m-cresol were placed in a 100 mL three-necked flask and were stirred at 180°C for 4 hours. After cooling to room temperature, the precipitated solid was filtered off and was washed with ethanol. The resulting solid was then recrystallized from dimethylformamide to obtain the target (B-3) (1.7 g, yield: 81%).

The resulting compound (B-3) was identified by ¹H-NMR.
¹H-NMR (DMSO-d6) δ (ppm): 8.49 (s, 2H), 8.11 (d, 4H), 7.78 (d, 4H)

### [Synthesis Example 2: Synthesis of Compound (B-31)]

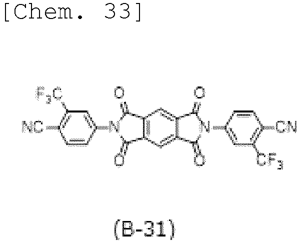

The same procedure as in Synthesis Example 1 was performed except that 1.2 g (10.5 mmol) of 4-aminobenzonitrile was replaced with 2.0 g (10.5 mmol) of 5-amino-2-cyanobenzotrifluoride to obtain the target (B-31) (2.3 g, yield: 83%).

The resulting compound (B-31) was identified by FD-MS and ¹H-NMR.
Mass spectrometry (FD-MS): 554
¹H-NMR (DMSO-d6) δ (ppm): 8.55 (s, 2H), 8.44 (d, 2H), 8.24 (d, 2H), 8.11 (d, 2H)

### [Synthesis Example 3: Synthesis of Compound (B-2)]

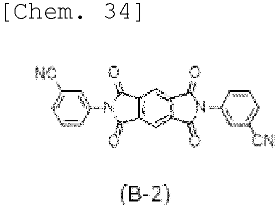

The same procedure as in Synthesis Example 1 was performed except that 1.2 g (10.5 mmol) of 4-aminobenzonitrile was replaced with 1.2 g (10.5 mmol) of 3-aminobenzonitrile to obtain the target (B-2) (1.5 g, yield: 71%).

The resulting compound (B-2) was identified by FD-MS.
Mass spectrometry (FD-MS): 418

### [Synthesis Example 4: Synthesis of Compound (B-103)]

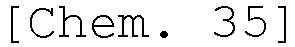

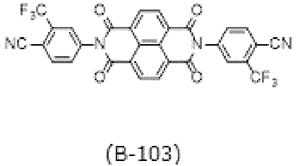

In a nitrogen stream, 1.3 g (5.0 mmol) of naphthalene-1,4,5,8-tetracarboxylic dianhydride, 1.9 g (10.5 mmol) of 5-amino-2-cyanobenzotrifluoride, 0.6 g (4.3 mmol) of isoquinoline and 20 mL of m-cresol were placed in a 100 mL three-necked flask and were stirred at 180°C for 5 hours. After cooling to room temperature, the precipitated solid was filtered off and was washed with ethanol. The resulting solid was then recrystallized from dimethylformamide to obtain the target (B-103) (1.7 g, yield: 56%).

The resulting compound (B-103) was identified by ¹H-NMR.
¹H-NMR (DMSO-d6) δ (ppm): 8.77 (s, 4H), 8.46 (d, 2H), 8.31 (s, 2H), 8.11 (d, 2H)

### [Synthesis Example 5: Synthesis of Compound (B-63)]

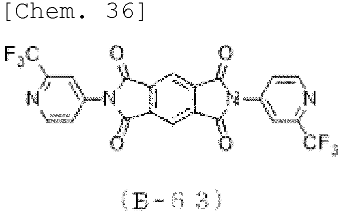

The same procedure as in Synthesis Example 1 was performed except that 1.2 g (10.5 mmol) of 4-aminobenzonitrile was replaced with 1.7 g (10.5 mmol) of 4-amino-2-(trifluoromethyl)pyridine to obtain the target (B-63) (1.4 g, yield: 55%).

The resulting compound (B-63) was identified by ¹H-NMR.
¹H-NMR (DMSO-d6) δ (ppm): 9.02 (d, 2H), 8.57 (s, 2H), 8.16 (d, 2H), 8.00 (d, 2H)

### [Synthesis Example 6: Synthesis of Compound (B-135)]

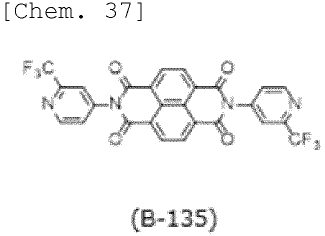

The same procedure as in Synthesis Example 4 was performed except that 1.9 g (10.5 mmol) of 5-amino-2-cyanobenzotrifluoride was replaced with 1.7 g (10.5 mmol) of 4-amino-2-(trifluoromethyl)pyridine to obtain the target (B-135) (1.8 g, yield: 64%).

The resulting compound (B-135) was identified by ¹H-NMR.
¹H-NMR (DMSO-d6) δ (ppm): 9.06 (d, 2H), 8.78 (s, 4H), 8.21 (s, 2H), 7.96 (d, 2H)

### [Synthesis Example 7: Synthesis of Compound (B-8)]

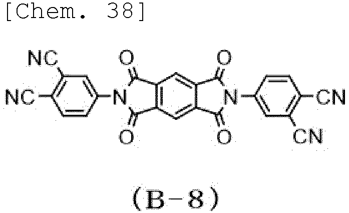

In a nitrogen stream, 1.1 g (5.0 mmol) of benzene-1,2,4,5-tetracarboxylic anhydride, 1.5 g (10.5 mmol) of 4-aminophthalonitrile, 0.6 g (4.3 mmol) of isoquinoline and 20 mL of m-cresol were placed in a 100 mL three-necked flask and were stirred at 140°C for 10 hours. After cooling to room temperature, the precipitated solid was filtered off and was washed with ethanol. The resulting solid was then recrystallized from dimethylformamide/toluene to obtain the target (B-8) (1.2 g, yield: 52%).

The resulting compound (B-8) was identified by ¹H-NMR.
¹H-NMR (DMSO-d6) δ (ppm): 8.57 (s, 2H), 8.38 (d, 2H), 8.30 (d, 2H), 8.11 (d, 2H)

### [Synthesis Example 8: Synthesis of Compound (B-67)]

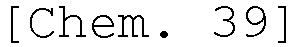

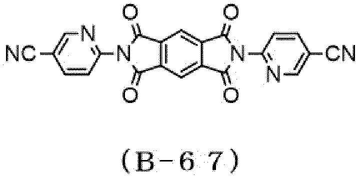

The same procedure as in Synthesis Example 7 was performed except that 1.5 g (10.5 mmol) of 4-aminophthalonitrile was replaced with 1.3 g (10.5 mmol) of 2-amino-5-cyanopyridine to obtain the target (B-67) (0.59 g, yield: 28%).

The resulting compound (B-67) was identified by FD-MS.
Mass spectrometry (FD-MS): 420

### [Synthesis Example 9: Synthesis of Compound (B-68)]

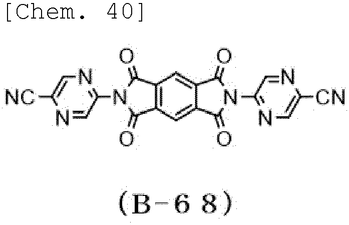

The same procedure as in Synthesis Example 7 was performed except that 1.5 g (10.5 mmol) of 4-aminophthalonitrile was replaced with 1.3 g (10.5 mmol) of 5-aminopyrazine-2-carbonitrile to obtain the target (B-68) (0.63 g, yield: 30%).

The resulting compound (B-68) was identified by ¹H-NMR.
¹H-NMR (DMSO-d6) δ (ppm): 9.40 (s, 2H), 9.07 (s, 2H), 8.62 (s, 2H)

### [Synthesis Example 10: Synthesis of Compound (B-177)]

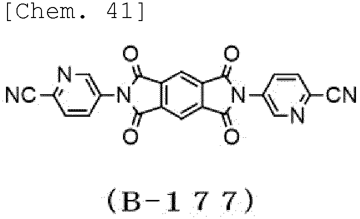

The same procedure as in Synthesis Example 7 was performed except that 1.5 g (10.5 mmol) of 4-aminophthalonitrile was replaced with 1.3 g (10.5 mmol) of 5-aminopicolinonitrile to obtain the target (B-177) (1.3 g, yield: 64%).

The resulting compound (B-177) was identified by ¹H-NMR.
¹H-NMR (DMSO-d6) δ (ppm): 8.96 (d, 2H), 8.57 (s, 2H), 8.32 (d, 2H), 8.26 (dd, 2H)

### [Synthesis Example 11: Synthesis of Compound (B-178)]

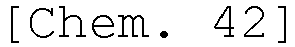

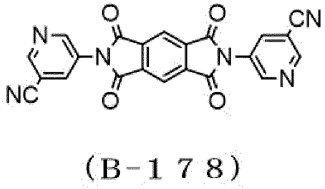

The same procedure as in Synthesis Example 7 was performed except that 1.5 g (10.5 mmol) of 4-aminophthalonitrile was replaced with 1.3 g (10.5 mmol) of 5-aminonicotinonitrile to obtain the target (B-178) (1.2 g, yield: 58%).

The resulting compound (B-178) was identified by ¹H-NMR.
¹H-NMR (DMSO-d6) δ (ppm): 9.16 (d, 2H), 9.06 (d, 2H), 8.59 (s, 2H), 8.52 (t, 2H)

### [Synthesis Example 12: Synthesis of Compound (B-187)]

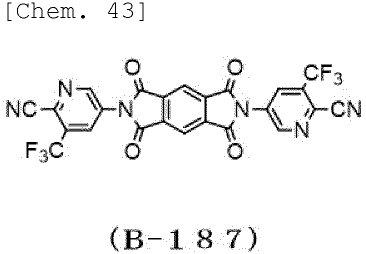

The same procedure as in Synthesis Example 7 was performed except that 1.5 g (10.5 mmol) of 4-aminophthalonitrile was replaced with 2.0 g (10.5 mmol) of 5-amino-3-(trifluoromethyl)picolinonitrile to obtain the target (B-187) (1.1 g, yield: 40%).

The resulting compound (B-187) was identified by FD-MS.
Mass spectrometry (FD-MS): 556

### [Synthesis Example 13: Synthesis of Compound (B-201)]

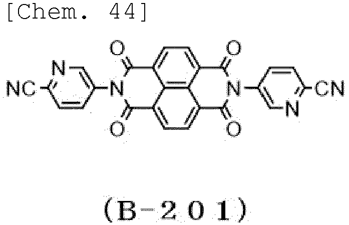

In a nitrogen stream, 1.3 g (5.0 mmol) of naphthalene-1,4,5,8-tetracarboxylic dianhydride, 1.3 g (10.5 mmol) of 5-aminopicolinonitrile, 0.6 g (4.3 mmol) of isoquinoline and 20 mL of m-cresol were placed in a 100 mL three-necked flask and were stirred at 140°C for 10 hours. After cooling to room temperature, the precipitated solid was filtered off and was washed with ethanol. The resulting solid was then recrystallized from dimethylformamide/toluene to obtain the target (B-201) (1.4 g, yield: 59%).

The resulting compound (B-201) was identified by ¹H-NMR.
¹H-NMR (DMSO-d6) δ (ppm): 8.92 (d, 2H), 8.78 (s, 4H), 8.34 (d, 2H), 8.27 (dd, 2H)

### [Synthesis Example 14: Synthesis of Compound (B-204)]

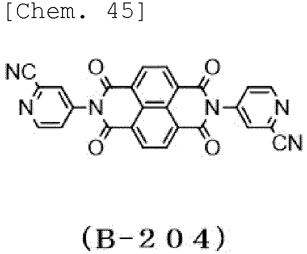

The same procedure as in Synthesis Example 13 was performed except that 1.3 g (10.5 mmol) of 5-aminopicolinonitrile was replaced with 1.3 g (10.5 mmol) of 4-aminopicolinonitrile to obtain the target (B-204) (0.94 g, yield: 40%).

The resulting compound (B-204) was identified by ¹H-NMR.
¹H-NMR (DMSO-d6) δ (ppm): 9.04 (d, 2H), 8.79 (s, 4H), 8.28 (d, 2H), 8.00 (dd, 2H)

### [Synthesis Example 15: Synthesis of Compound (B-209)]

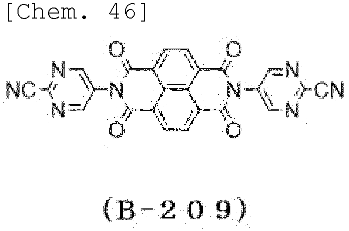

The same procedure as in Synthesis Example 13 was performed except that 1.3 g (10.5 mmol) of 5-aminopicolinonitrile was replaced with 1.3 g (10.5 mmol) of 5-aminopyrimidine-2-carbonitrile to obtain the target (B-209) (1.2 g, yield: 50%).

The resulting compound (B-209) was identified by FD-MS.
Mass spectrometry (FD-MS): 472

### [Synthesis Example 16: Synthesis of Compound (B-258)]

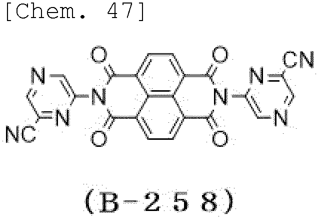

The same procedure as in Synthesis Example 13 was performed except that 1.3 g (10.5 mmol) of 5-aminopicolinonitrile was replaced with 1.3 g (10.5 mmol) of 2-amino-6-cyanopyrazine to obtain the target (B-258) (0.35 g, yield: 15%).

The resulting compound (B-258) was identified by ¹H-NMR.
¹H-NMR (DMSO-d6) δ (ppm): 9.48 (s, 2H), 9.30 (s, 2H), 8.81 (br-s, 4H)

### <Fabrication 1 and Evaluation 1 of Hole-Only Device>

### [Element Example 1]

A hole-only device was fabricated and evaluated for its hole transport characteristics. The hole-only device had the following structure: first electrode/first hole injection layer/hole transport layer/hole transport promoting layer/second electrode.

### (First Electrode)

As a substrate having a first electrode on a surface thereof, a glass substrate with an ITO transparent electrode made of a striped pattern of ITO film (thickness: 110 nm) was provided. This substrate was cleaned with isopropyl alcohol and was then surface-treated by ultraviolet-ozone cleaning.

### (Preparation for Vacuum Deposition)

Of the two surfaces of the surface-treated substrate, the individual layers were formed on top of each other on the surface with the ITO film thereon by vacuum deposition.

First, the glass substrate was introduced into a vacuum deposition chamber, and the pressure therein was reduced to 1.0 × 10⁻⁴ Pa. The individual layers were then formed under their respective film deposition conditions in the following order.

### (Formation of First Hole Injection Layer)

MoO₃ was deposited to a thickness of 1 nm on the ITO film to form a hole injection layer.

### (Formation of Hole Transport Layer)

(HTL-1) was deposited as a hole transport material to a thickness of 100 nm to form a hole transport layer. (HTL-1) was synthesized by a method described in Japanese Unexamined Patent Application Publication No. 2018-193371.

### (Formation of Hole Transport Promoting Layer)

A compound (B-3) purified by sublimation was deposited to a thickness of 10 nm to form a hole transport promoting layer.

### (Formation of Second Electrode)

Au was deposited to a thickness of 80 nm to form a second electrode.

### (Hole Transport Ability Evaluation of Hole-Only Device)

A positive electric field and a negative electric field were applied to the first electrode and the second electrode, respectively, of the hole-only device of Element Example 1, and the voltage value was measured at a current density of 10 mA/cm². The results are shown in Table 1.

### [Element Example 2]

A hole-only device was fabricated in the same manner as in Element Example 1 except that the compound (B-3) used to form the hole transport promoting layer in Example 1 was replaced with the compound (B-31). The hole transport ability of the resulting hole-only device was evaluated in the same manner as in Element Example 1. The results are shown in Table 1.

### [Element Example 3]

A hole-only device was fabricated in the same manner as in Element Example 1 except that the compound (B-3) used to form the hole transport promoting layer in Example 1 was replaced with the compound (B-63). The hole transport ability of the resulting hole-only device was evaluated in the same manner as in Element Example 1. The results are shown in Table 1.

### [Element Example 4]

A hole-only device was fabricated in the same manner as in Element Example 1 except that the compound (B-3) used to form the hole transport promoting layer in Example 1 was replaced with the compound (B-68). The hole transport ability of the resulting hole-only device was evaluated in the same manner as in Element Example 1. The results are shown in Table 1.

### [Element Example 5]

A hole-only device was fabricated in the same manner as in Element Example 1 except that the compound (B-3) used to form the hole transport promoting layer in Example 1 was replaced with the compound (B-178). The hole transport ability of the resulting hole-only device was evaluated in the same manner as in Element Example 1. The results are shown in Table 1.

### [Element Example 6]

A hole-only device was fabricated in the same manner as in Element Example 1 except that the compound (B-3) used to form the hole transport promoting layer in Example 1 was replaced with the compound (B-201). The hole transport ability of the resulting hole-only device was evaluated in the same manner as in Element Example 1. The results are shown in Table 1.

### [Comparative Element Example 1]

A hole-only device was fabricated in the same manner as in Element Example 1 except that the hole-only device included no hole transport promoting layer. The hole transport ability of the resulting hole-only device was evaluated in the same manner as in Element Example 1. The results are shown in Table 1.

### [Comparative Element Example 2]

A hole-only device was fabricated in the same manner as in Element Example 1 except that the compound (B-3) used to form the hole transport promoting layer in Example 1 was replaced with the compound (NPT). The hole transport ability of the resulting hole-only device was evaluated in the same manner as in Element Example 1. The results are shown in Table 1.

**[Table 1]**

| | Hole transport material | Hole transport promoting material | Voltage (V) |
|---|---|---|---|
| Element Example 1 | HTL-1 | B-3 | 2.51 |
| Element Example 2 | HTL-1 | B-31 | 2.77 |
| Element Example 3 | HTL-1 | B-63 | 1.01 |
| Element Example 4 | HTL-1 | B-68 | 1.98 |
| Element Example 5 | HTL-1 | B-178 | 2.15 |
| Element Example 6 | HTL-1 | B-201 | 2.33 |
| Comparative Element Example 1 | HTL-1 | None | 4.70 |
| Comparative Element Example 2 | HTL-1 | NPT | 3.90 |

The elements of Element Example 1, in which the compound (B-3), the compound (B-31), the compound (B-63), the compound (B-68), the compound (B-178) and the compound (B-201) were used as hole transport promoting materials (their respective HOD voltages were 2.51 V, 2.77 V, 1.01 V, 1.98 V, 2.15 V and 2.33 V), exhibited lower HOD voltages than the element of Comparative Element Example 1, in which no hole transport promoting material was used (the HOD voltage was 4.70 V). In addition, the elements of Element Example 1, in which the compound (B-3), the compound (B-31), the compound (B-63), the compound (B-68), the compound (B-178) and the compound (B-201) were used as hole transport promoting materials (their respective HOD voltages were 2.51 V, 2.77 V, 1.01 V, 1.98 V, 2.15 V and 2.33 V), exhibited lower HOD voltages than the element of Comparative Element Example 2, in which a known material, namely, NPT, was used (the HOD voltage was 3.90 V).

### <Fabrication and Evaluation of Photoelectric Conversion Element>

### [Element Example 7]

A photoelectric conversion element 1 was fabricated and evaluated for its dark current and external quantum efficiency. The photoelectric conversion element 1 had the following multilayer configuration: substrate/second electrode 16/electron transport layer 15/light-receiving layer 14/hole transport layer 13/hole transport promoting layer 12/first electrode 11.

### (Provision of Substrate and Second Electrode 16)

As a substrate having a second electrode on a surface thereof, a glass substrate with an indium tin oxide (ITO) transparent electrode made of a striped pattern of ITO film (thickness: 110 nm) having a width of 2 mm was provided. This substrate was cleaned with isopropyl alcohol and was then surface-treated by ultraviolet-ozone cleaning.

### (Preparation for Vacuum Deposition)

The individual layers were formed on top of each other on the cleaned and surface-treated substrate by vacuum deposition.

First, the glass substrate was introduced into a vacuum deposition chamber, and the pressure therein was reduced to 7.0 × 10⁻⁵ Pa. The individual layers were then formed under their respective film deposition conditions in the following order.

### (Formation of Electron Transport Layer 15)

A compound purified by sublimation, namely, 4,6-bis(3,5-di(pyridin-4-yl)phenyl)-2-methylpyrimidine, was deposited to a thickness of 10 nm at a rate of 0.03 nm/second to form an electron transport layer 15.

### (Formation of Light-Receiving Layer 14)

N,N-dimethylquinacridone and fullerene C60 were deposited to a thickness of 250 nm in a ratio of 4:1 (mass ratio) to form a photoelectric conversion layer 14. The deposition rate was 0.13 nm/second.

### (Formation of Hole Transport Layer 13)

(HTL-1) was deposited as a hole transport material to a thickness of 10 nm at a rate of 0.10 nm/second to form a hole transport layer 13.

### (Formation of Hole Transport Promoting Layer 12)

The compound (B-3) was deposited to a thickness of 10 nm at a rate of 0.20 nm/second to form a hole transport promoting layer 12.

### (Formation of First Electrode 11)

Finally, a metal mask was placed perpendicular to the ITO stripes on the substrate, and a first electrode 11 was deposited thereon. As the first electrode, Au was deposited to a thickness of 80 nm. The deposition rate of Au was 0.1 nm/second.

As described above, a photoelectric conversion element 1 illustrated in Fig. 1 with an area of 4 mm² was fabricated. The photoelectric conversion element fabricated as described above was evaluated for its current in a dark place (dark current) and external quantum efficiency by applying a voltage of 2.5 V in absolute value such that electrons were transported to the second electrode 16 side and holes were transported to the first electrode 11 side. The dark current was measured by evaluation using a Keithley 2636B source measure unit. The external quantum efficiency was measured using a solar cell spectral sensitivity measurement instrument (manufactured by Soma Opt Ltd.). The measurement was performed with irradiation light at a wavelength of 560 nm and an intensity of 50 µW/cm². The results are shown in Table 2. The dark current and the external quantum efficiency are relative values with those of Comparative Element Example 3 described later being reference values (100). A lower value of dark current indicates higher performance, and a higher value of external quantum efficiency indicates higher performance.

### [Element Example 8]

A photoelectric conversion element of Element Example 8 was fabricated in the same manner as in Element Example 7 except that the compound (B-3) was replaced with the compound (B-31) in the formation of the hole transport promoting layer 12. The dark current and the external quantum efficiency were measured in the same manner as in Element Example 7. The results are shown in Table 2.

### [Element Example 9]

A photoelectric conversion element of Element Example 9 was fabricated in the same manner as in Element Example 7 except that the compound (B-3) was replaced with the compound (B-63) in the formation of the hole transport promoting layer 12. The dark current and the external quantum efficiency were measured in the same manner as in Element Example 7. The results are shown in Table 2.

### [Element Example 10]

A photoelectric conversion element of Element Example 10 was fabricated in the same manner as in Element Example 7 except that the compound (B-3) was replaced with the compound (B-135) in the formation of the hole transport promoting layer 12. The dark current and the external quantum efficiency were measured in the same manner as in Element Example 7. The results are shown in Table 2

### [Element Example 11]

A photoelectric conversion element of Element Example 11 was fabricated in the same manner as in Element Example 7 except that the compound (B-3) was replaced with the compound (B-8) in the formation of the hole transport promoting layer 12. The dark current and the external quantum efficiency were measured in the same manner as in Element Example 7. The results are shown in Table 2.

### [Element Example 12]

A photoelectric conversion element of Element Example 12 was fabricated in the same manner as in Element Example 7 except that the compound (B-3) was replaced with the compound (B-68) in the formation of the hole transport promoting layer 12. The dark current and the external quantum efficiency were measured in the same manner as in Element Example 7. The results are shown in Table 2.

### [Element Example 13]

A photoelectric conversion element of Element Example 13 was fabricated in the same manner as in Element Example 7 except that the compound (B-3) was replaced with the compound (B-177) in the formation of the hole transport promoting layer 12. The dark current and the external quantum efficiency were measured in the same manner as in Element Example 7. The results are shown in Table 2.

### [Element Example 14]

A photoelectric conversion element of Element Example 14 was fabricated in the same manner as in Element Example 7 except that the compound (B-3) was replaced with the compound (B-178) in the formation of the hole transport promoting layer 12. The dark current and the external quantum efficiency were measured in the same manner as in Element Example 7. The results are shown in Table 2.

### [Element Example 15]

A photoelectric conversion element of Element Example 15 was fabricated in the same manner as in Element Example 7 except that the compound (B-3) was replaced with the compound (B-187) in the formation of the hole transport promoting layer 12. The dark current and the external quantum efficiency were measured in the same manner as in Element Example 7. The results are shown in Table 2.

### [Element Example 16]

A photoelectric conversion element of Element Example 16 was fabricated in the same manner as in Element Example 7 except that the compound (B-3) was replaced with the compound (B-204) in the formation of the hole transport promoting layer 12. The dark current and the external quantum efficiency were measured in the same manner as in Element Example 7. The results are shown in Table 2.

### [Element Example 17]

A photoelectric conversion element of Element Example 17 was fabricated in the same manner as in Element Example 7 except that the compound (B-3) was replaced with the compound (B-209) in the formation of the hole transport promoting layer 12. The dark current and the external quantum efficiency were measured in the same manner as in Element Example 7. The results are shown in Table 2.

### [Comparative Element Example 3]

A photoelectric conversion element of Comparative Element Example 3 was fabricated in the same manner as in Element Example 7 except that the compound (B-3) was replaced with the compound (NPT) in the formation of the hole transport promoting layer 12. The dark current and the external quantum efficiency were measured in the same manner as in Element Example 7. The results are shown in Table 2.

### [Comparative Element Example 4]

A photoelectric conversion element of Comparative Element Example 4 was fabricated in the same manner as in Element Example 7 except that no hole transport promoting layer 12 was formed. The dark current and the external quantum efficiency were measured in the same manner as in Element Example 7. The results are shown in Table 2.

**[Table 2]**

| | Hole transport material | Hole transport promoting material | Dark current (A/cm²) | External quantum efficiency |
|---|---|---|---|---|
| Element Example 7 | HTL-1 | B-3 | 59 | 105 |
| Element Example 8 | HTL-1 | B-31 | 7 | 103 |
| Element Example 9 | HTL-1 | B-63 | 13 | 106 |
| Element Example 10 | HTL-1 | B-135 | 19 | 103 |
| Element Example 11 | HTL-1 | B-8 | 12 | 104 |
| Element Example 12 | HTL-1 | B-68 | 30 | 104 |
| Element Example 13 | HTL-1 | B-177 | 49 | 105 |
| Element Example 14 | HTL-1 | B-178 | 14 | 103 |
| Element Example 15 | HTL-1 | B-187 | 8 | 104 |
| Element Example 16 | HTL-1 | B-204 | 15 | 103 |
| Element Example 17 | HTL-1 | B-209 | 24 | 103 |
| Comparative Element Example 3 | HTL-1 | NPT | 100 | 100 |
| Comparative Element Example 4 | HTL-1 | None | 7200 | N/A |

As shown in Table 2, the elements of the Element Examples, in which materials for photoelectric conversion elements for image capture elements according to the present invention were used, exhibited lower dark currents and higher external quantum efficiencies than the elements of the Comparative Element Examples.

Because the organic electronic element of the present invention contains the compound having the partial structure represented by formula (1) above, more specifically, the compound represented by formula (2) above, the organic electronic element has an improved hole transport ability and can thus perform more efficient photoelectric change when used as a photoelectric conversion element. In addition, because the organic electronic element of the present invention contains the compound having the partial structure represented by formula (1) above, more specifically, the compound represented by formula (2) above, the organic electronic element can exhibit low dark current and high external quantum efficiency.

### Reference Signs List

- 1.: photoelectric conversion element
- 11.: first electrode
- 12.: hole transport promoting layer
- 13.: hole transport layer
- 14.: light-receiving layer
- 15.: electron transport layer
- 16.: second electrode

## Claims

1. An organic electronic element comprising a first electrode, a second electrode and an organic layer disposed between the first electrode and the second electrode,
wherein the organic layer includes a hole transport layer and a hole transport promoting layer containing a compound having a partial structure represented by the following formula (1), or includes a layer comprising a mixture of a hole transport material and the compound having the partial structure represented by formula (1), and
the organic electronic element includes a light-receiving layer: 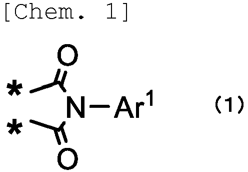 (wherein * represents a bond, and formula (1) forms a cyclic imide structure;
Ar¹ represents a monocyclic or fused-ring aromatic hydrocarbon group optionally substituted with a substituent or a monocyclic or fused-ring heteroaromatic group optionally substituted with a substituent, the aromatic hydrocarbon group may be a group in which a plurality of aromatic hydrocarbon groups are linked together directly or with a linking group therebetween, the heteroaromatic group may be a group in which a plurality of heteroaromatic groups are linked together directly or with a linking group therebetween, and Ar¹ may be a group in which the aromatic hydrocarbon group and the heteroaromatic group are linked together; and
the substituent with which the aromatic hydrocarbon group or the heteroaromatic group is optionally substituted is a cyano group, a fluoro group, a chloro group, a bromo group, an iodo group, a trifluoromethyl group, a methyl group, a fluoroalkyl group having 2 to 10 carbon atoms, a fluoroalkoxy group having 1 to 10 carbon atoms or an alkyl group having 2 to 10 carbon atoms).

2. The organic electronic element according to Claim 1, wherein the hole transport layer and the hole transport promoting layer are disposed adjacent to each other between the first electrode and the second electrode.

3. The organic electronic element according to Claim 1, wherein the compound having the partial structure represented by formula (1) is a compound represented by the following formula (2): 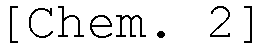 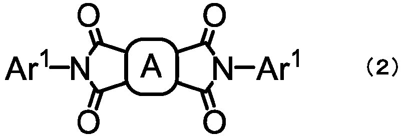 (wherein
Ar¹ represents the same group as Ar¹ in formula (1), and one Ar¹ group is the same group as another Ar¹ group; and
a ring A represents a monocyclic or fused-ring aromatic hydrocarbon ring, and the aromatic hydrocarbon ring may be a ring in which a plurality of aromatic hydrocarbon rings are linked together directly or with a linking group therebetween).

4. The organic electronic element according to Claim 3, wherein the ring A in formula (2) is represented by any of the following (A-1) to (A-9):

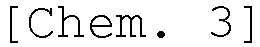

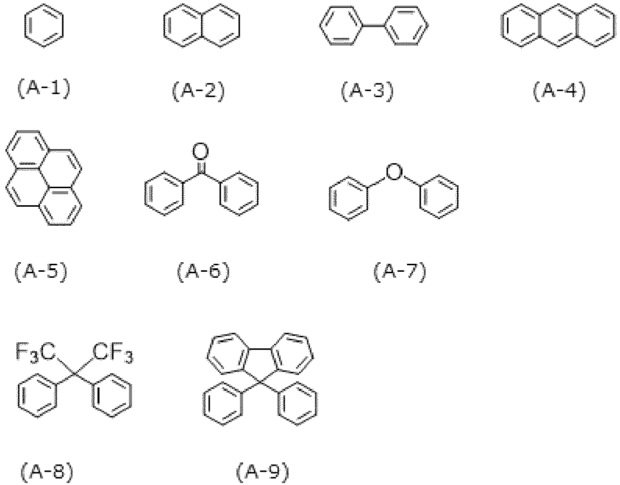

5. The organic electronic element according to Claim 4, wherein the ring A in formula (2) is formula (A-1) or formula (A-2).

6. The organic electronic element according to Claim 1, wherein Ar¹ in formula (1) is an electron-withdrawing substituent.

7. The organic electronic element according to Claim 1, wherein Ar¹ in formula (1) is a phenyl group, a pyridyl group, a pyrazyl group or a pyrimidyl group, each substituted with at least one group selected from the group consisting of a cyano group, a fluoro group and a trifluoromethyl group.

8. An imide compound represented by the following formula (3) : 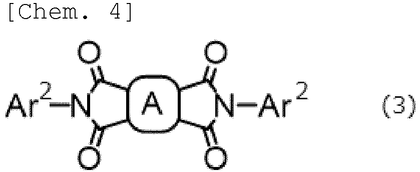 (wherein
Ar² represents a pyridyl group, a pyrazyl group or a pyrimidyl group, each substituted with at least one group selected from the group consisting of a cyano group, a fluoro group and a trifluoromethyl group, and one Ar² group is the same group as another Ar² group; and
a ring A represents any of the following (A-1) to (A-9) :

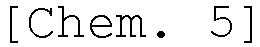

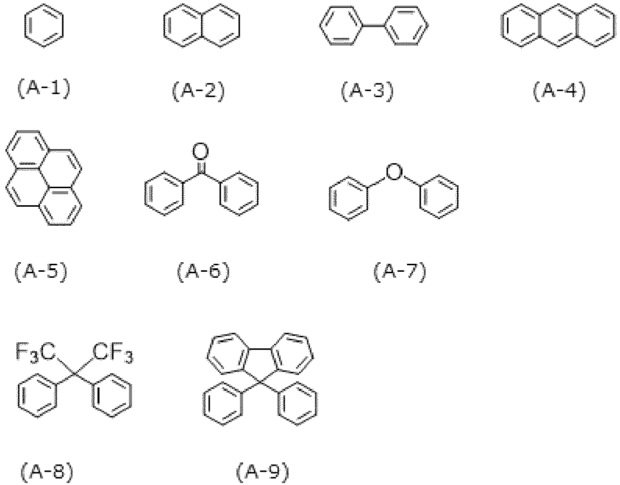

9. The imide compound according to Claim 8, wherein the ring A in formula (3) is formula (A-1) or formula (A-2).
